(19) Europäisches Patentamt
European Patent Office
Office européen des brevets

(11) **EP 3 960 206 B1**

(12) **EUROPEAN PATENT SPECIFICATION**

(45) Date of publication and mention
of the grant of the patent:
**19.02.2025 Bulletin 2025/08**

(51) International Patent Classification (IPC):
*A61L 2/00* *(2006.01)*    *A61L 2/10* *(2006.01)*
*C03C 3/089* *(2006.01)*    *C03C 4/00* *(2006.01)*
*C02F 1/00* *(2023.01)*    *G01N 30/00* *(2006.01)*

(21) Application number: **21191643.2**

(22) Date of filing: **17.08.2021**

(52) Cooperative Patent Classification (CPC):
**A61L 2/10; A61L 2/0047; A61L 9/20; C02F 1/325;**
**C03C 3/089; C03C 3/118; C03C 4/0085;**
A61L 2202/11; A61L 2202/25; C02F 2201/3222;
Y02W 10/37

(54) **METHOD FOR ERADICATING METHICILLIN-RESISTANT STAPHYLOCOCCUS AUREUS**

VERFAHREN ZUR AUSROTTUNG VON METHICILLIN-RESISTENTEM STAPHYLOCOCCUS AUREUS

PROCÉDÉ D'ÉRADICATION DE STAPHYLOCOCCUS AUREUS RÉSISTANT À LA MÉTHICILLINE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **03.12.2020 EP 20211687**

(43) Date of publication of application:
**02.03.2022 Bulletin 2022/09**

(73) Proprietor: **SCHOTT AG**
**55122 Mainz (DE)**

(72) Inventors:
• **Petershans, André**
  **92507 Nabburg (DE)**
• **Megges, Klaus**
  **92637 Weiden i. d. Oberpfalz (DE)**
• **Krüger, Susanne**
  **55127 Mainz (DE)**
• **Pfeiffer, Thomas**
  **55218 Ingelheim (DE)**

(74) Representative: **Fuchs Patentanwälte**
**Partnerschaft mbB**
**Tower 185**
**Friedrich-Ebert-Anlage 35-37**
**60327 Frankfurt am Main (DE)**

(56) References cited:
WO-A1-2008/029799    CN-A- 110 240 402
DE-C2- 3 320 980    JP-A- S6 287 433
US-A- 5 045 509

• **NARITA KOUJI ET AL: "Disinfection and healing effects of 222-nm UVC light on methicillin-resistantStaphylococcus aureusinfection in mouse wounds", JOURNAL OF PHOTOCHEMISTRY AND PHOTOBIOLOGY B: BIOLOGY, ELSEVIER SCIENCE S.A., BASEL, CH, vol. 178, 27 October 2017 (2017-10-27), pages 10 - 18, XP085337269, ISSN: 1011-1344, DOI: 10.1016/J.JPHOTOBIOL.2017.10.030**
• **KAIKI YUKI ET AL: "Methicillin-resistant Staphylococcus aureus contamination of hospital-use-only mobile phones and efficacy of 222-nm ultraviolet disinfection", vol. 000, 10 November 2020 (2020-11-10), AMSTERDAM, NL, pages 1 - 4, XP055797466, ISSN: 0196-6553, Retrieved from the Internet <URL:https://www.ajicjournal.org/article/S0196-6553(20)30993-7/pdf> DOI: 10.1016/j.ajic.2020.11.011**

EP 3 960 206 B1

**Description**

The present invention relates to methods for eradication of Methicillin-resistant *Staphylococcus aureus* (MRSA), especially to methods for eradicating MRSA from UV-sensitive surfaces. The method comprises the use of germicidal UV light within the wavelength range of from 207 nm to 222 nm, wherein the UV light is emitted by a UV lamp having a lamp cover made of specific UV-transparent borosilicate glasses. The present invention includes UV-transparent glasses, uses of such UV-transparent glasses,

Background of the invention

Methicillin-resistant *Staphylococcus aureus* (MRSA) refers to a group of Gram-positive bacteria that are genetically distinct from other strains of *Staphylococcus aureus.* MRSA is responsible for several difficult-to-treat infections in humans. MRSA is any strain of *S. aureus* that has developed multiple drug resistance to beta-lactam antibiotics through horizontal gene transfer and/or natural selection. β-lactam antibiotics are a broad-spectrum group that include some penams (penicillin derivatives such as methicillin and oxacillin) and cephems such as the cephalosporins.
MRSA is common in hospitals, prisons, and nursing homes, where people with open wounds, invasive devices such as catheters, and weakened immune systems are at a greater risk of hospital-acquired infection. MRSA began as a hospital-acquired infection, but has become community-acquired, as well as livestock-acquired.
In recent studies MRSA could be identified in many surface waters, such as rivers and lakes, increasing the chances that MRSA will contaminate drinking water sources and can easily be transmitted to all kinds of sensitive areas, such as nursery schools, schools, hospitals, special-care homes, retirement homes and other health-care facilities.
This is especially dangerous, since common cleaning systems of sewage and sewage treatment plants are currently not always sufficiently equipped to filter or to eradicate multi-resistant germs out of the water.
One of the few countries not yet overwhelmed by MRSA is the Netherlands. An important part of the success of the Dutch strategy was the attempt to eradicate MRSA in patients before being dismissed from the hospital.
Thus, there is a great need of easy-to-use and effective eradication methods against MRSA, especially MRSA that resides on surfaces sensitive to conventional sterilization methods.
Methods for eradication of MRSA by light have been reported in the art., for instance from NARITA KOUJI ET AL: "Disinfection and healing effects of 222-nm UVC light on methicillin-resistantStaphylococcus aureusinfection in mouse wounds" and KAIKI YUKI ET AL: "Methicillin-resistant Staphylococcus aureus contamination of hospital-use-only mobile phones and efficacy of 222-nm ultraviolet disinfection". The so called "photosensitizer-method" is used widely in hospitals and other areas with elevated MRSA-risks (or other bacterial contaminations). This method makes use of photosensitizers, mostly dye molecules, that become excited when illuminated with light. When excited by light, these molecules produce reactive oxygen species, which then eradicate the bacteria.
However, not all reported methods using photosensitizers are sufficient to eradicate enough microorganisms to prevent infections effectively. This is because photosensitizers may not be concentrated enough to do significant damage. In addition, many photosensitizers are hydrophobic. This makes it difficult to disperse them in aqueous environments, where microorganisms typically exist (e.g. biofilms).
Another method in the art is called "ultraviolet germicidal irradiation" (UVGI), that uses short-wavelength ultraviolet (UVC) light to kill or inactivate microorganisms by destroying nucleic acids and disrupting their DNA, leaving them unable to perform vital cellular functions. UVGI is used in a variety of applications, such as food, air, and water purification.
UVGI devices can produce strong enough UVC light in circulating air or water systems to make them inhospitable environments to microorganisms such as bacteria, viruses, molds and other pathogens. UVGI can be coupled with a filtration system to sanitize air and water. The application of UVGI to disinfection has been an accepted practice since the mid-20th century. It has been used primarily in medical sanitation and sterile work facilities.
Increasingly, it has been employed to sterilize drinking and wastewater, as the holding facilities are enclosed and can be circulated to ensure a higher exposure to the UV. In recent years UVGI has found renewed application in air purifiers. Existing UVGI-methods use UV-light in the wavelength of around 250 nm, for example conventional germicidal UV lamps based on mercury-vapor lamps, radiate with a wavelength of 254 nm.
However, it has been reported that conventional germicidal UV lamps are harmful for the eye, produce pre-mutagenic UV-associated DNA lesions in human skin and are cytotoxic to exposed mammalian skin.
Thus, the danger of damaging effects, including even the induction of cancer or other mutagenic diseases, prevent the direct use of common UVGI-methods for eradication of MRSA on mammalian skin, such as for example the skin of patients, health care personnel or livestock.

Description of the invention

It has been recently reported that far-UVC-light kills bacteria efficiently regardless of their drug-resistant proficiency, but without the skin- or eye-damaging effects associated with conventional germicidal UV exposure.

However, UV-absorption of UV-light at wavelengths of about 200 nm to about 250 nm is very high in otherwise transparent covers. For example, UV-light does not transmit well through conventional glasses at wavelengths beyond 320 nm. Conventional borosilicate glasses do not transmit light at wavelengths below 290 nm. Thus, these covers have the disadvantage that they are either impermissible for far-UV-light, or at least a high amount of energy is necessary to guarantee a sufficient UV-exposure of the treated surface, e.g. the treated skin. This high operational energy again results in significant energy dissipation and increases thermal stress to the cover, as well as to the whole device. The result is a reduced lifetime and increased maintenance cost of the device. CN 110 240 402 A, US 5 045 509 A, JP S62 87433 A disclose UV transparent borosilicate glasses.

Thus, when assessing prior art methods, the problem was to provide a new UVGI-method, which allows the application of UV in the far UVC in order to facilitate direct MRSA-treatment of sensitive surfaces, such as mammalian skin or other materials that are UV-sensitive, such as certain gasses and/or liquids.

The problem is solved by the method according to claim 1 and a glass according to claim 7.

UV radiation is able to split organic bonds. As a result, it is hostile to life by destroying biogenic substances. In addition, many plastics are damaged by ultraviolet radiation due to haze, embrittlement, and/or decay. Thus, UV-light can be harmful to a number of sensitive surfaces or other materials, which may be UV-sensitive, such as certain gasses and/or liquids.

In humans, excessive exposure to UV radiation can result in acute and chronic harmful effects on the eye's dioptric system and retina. The skin, the circadian and immune systems can also be affected. The skin and eyes are most sensitive to damage by UV at 265 to 275 nm.

Artificial UVC light of wavelengths around 250 nm, as being radiated for example by conventional UVGI-lamps, such as for example mercury-vapor lamps, produce pre-mutagenic UV-associated DNA lesions for example in human skin models and are cytotoxic to exposed mammalian skin. The eye is most sensitive to damage by UV in the lower UVC band at 265 nm to 275 nm. Radiation of this wavelength is almost absent from sunlight but is found in welder's arc lights and other artificial sources. Exposure to these can cause "welder's flash" or "arc eye" (photokeratitis) and can lead to cataracts, pterygium and pinguecula formation.

Thus, the wavelengths, which are applied according to the inventive method, are in the range of from 207 nm to 222 nm. Ultraviolet (UV) light of about 207 nm has similar antimicrobial properties as typical germicidal UV light (254 nm), but without inducing damage to outer tissue coverings of higher animals, such as amphibian, reptile, bird, mammal or human skin. However, in other embodiments it may also be used to eradicate MRSA from the outer surface of mollusks (shells) and/or arthropods (exoskeleton).

The limited penetration distance of 207 nm light in biological samples (e.g. *stratum corneum*) compared with that of 254 nm light allows for the selective antimicrobial treatment without harming the treated surface, especially mammalian or human skin, such as the skin of a patient or health care professionals.

Considering the eye, the most important target from the perspective of UV risk is the lens. The lens is located distal to the cornea, which is sufficiently thick (500 $\mu$m) such that penetration of 200 nm light through the cornea to the lens is essentially zero. Even if one considers effects on the cornea from the perspective of photokeratitis, any protective device against eye splash, which is now almost universal amongst surgical staff, would be expected to fully protect the cornea from 207 nm UV exposure.

The proposed bactericidal application of 207 nm UV light in the presence of humans is based on the fact that UV light at a wavelength of around 200 nm is very strongly absorbed by proteins (particularly through the peptide bond) and other biomolecules, so its ability to penetrate biological material is very limited. Thus, for example the intensity of 200 nm UV light is reduced by half in only about 0.3 mm of tissue, compared with about 3 mm at 250 nm and much longer distances for higher UV wavelengths. By contrast, 200 nm UV light is only minimally absorbed in water.

At the cellular level, bacteria are much smaller than almost any human cell. Typical bacterial cells are less than 1 $\mu$m in diameter, whereas typical eukaryotic cells range in diameter from about 10 to 25 $\mu$m.

It follows, that 200 nm UV light can penetrate throughout typical bacteria-cells but cannot penetrate significantly beyond the outer perimeter of the cytoplasm of typical eukaryotic cells, such as human cells, and will be drastically attenuated before reaching the eukaryotic cell nucleus.

By contrast, higher wavelength light from a conventional germicidal lamp can reach human cell nuclei without major attenuation. Based on these biophysical considerations, while radiation from a conventional UVC lamp is cytotoxic and mutagenic to both bacteria and human cells, 200 nm UV light is cytotoxic to bacteria, but much less cytotoxic or mutagenic to human cells.

UV-light of wavelengths significant below 200 nm, however, are not useful, because at these wavelengths a sufficient eradication of MRSA cannot be reached anymore. Furthermore, at wavelengths below 200 nm, UV reacts with oxygen and forms ozone, an effect which is not desired.

The UVC light in the range of about 207 to 222 nm eradicates bacteria efficiently regardless of their drug-resistant proficiency, but without the skin and eye damaging effects associated with conventional germicidal UV exposure.

The term "eradication" is used herein for any reduction of MRSA after treatment of more than 90%, more than 95%, more than 99%, more than 99.9%, or more than 99.99% according to ISO 22196:2011-08-31.

In order to achieve such an eradication, in one embodiment the invention pertains to a method wherein the UV-exposure of the MRSA and/or the surface to be treated is in the range from 2,000 to 8,000 $\mu W \cdot s/cm^2$, from 2,100 to 7,000 $\mu W \cdot s/cm^2$, from 2,200 to 5,000 $\mu W \cdot s/cm^2$, or from 2,300 to 3,000 $\mu W \cdot s/cm^2$. In one embodiment, the UV-exposure of at least about 2,500 $\mu W \cdot s/cm^2$ results in a 90% reduction of MRSA.

The methods of the present invention can be used for eradicating MRSA on all kinds of UV-sensitive material, which is sensitive to conventional UV-radiation of wavelengths above 222 nm. Such "UV-sensitive materials" may be any material where UV of wavelengths above 222 nm, above 250 nm and/or up to 295 nm is able to split organic or inorganic bonds. Such UV-sensitive material may include any plastics, which are damaged by ultraviolet radiation between 222 nm and/or up to 295 nm due to haze, embrittlement, and/or decay.

In another embodiment, the UV-sensitive material may be susceptible to crosslinking of monomers to produce specific polymers by UV-radiation above 222 nm, or above 250 nm and/or up to 295 nm. In yet another embodiment the UV-sensitive material may be a gas or a liquid, which is sensitive to UV above 222 nm, or above 250 nm and/or up to 295 nm.

In yet another embodiment, the UV-sensitive material may be a pharmaceutical composition, which is sensitive to UV above 222 nm, or above 250 nm and/or up to 295 nm.

In yet another embodiment, the UV-sensitive material may be a biological tissue surface, such as a skin of an insect, invertebrate, vertebrate, mammal or human, (e.g. mollusc, fish, amphibia, reptile, bird, mammal and/or human or a chitinous exoskeleton from an arthropod, such as a lobster or an insect).

Thus, the term "biological tissue surface" according to the definition of this invention encompasses all biological surfaces, which may be harmed by UV-radiation above 222 nm, or above 250 nm and/or up to 295 nm. In one embodiment, the invention includes biological surfaces which may be harmed by UV-radiation outside the wavelength range of 207 to 222 nm, and which are not harmed by UV-radiation within the wavelength range of 207 to 222 nm.

Within this invention the term "tissue" is used for any cellular organizational level between cells and a complete organ. A tissue is an ensemble of similar cells and their extracellular matrix from the same origin that together carry out a specific function. Organs are then formed by the functional grouping together of multiple tissues.

Of course, especially tissue, which may be exposed to UV-radiation during an MRSA-eradication method should be encompassed. In most of the cases, those tissues will be epithelial tissues that are formed by cells that cover the organ surfaces, such as the surface of skin, the airways, the reproductive tract, and the inner lining of the digestive tract. The cells comprising an epithelial layer are linked via semi-permeable, tight junctions; hence, this tissue provides a barrier between the external environment and the organ it covers. In addition to this protective function, epithelial tissue may also be specialized to function in secretion, excretion and absorption. Epithelial tissue helps to protect organs from microorganisms, injury, and fluid loss.

Thus, methods of this invention include preferably those methods where conventional UVGI-methods may not be applicable or suitable, such as for example UV-treatment of MRSA residing on a UV-sensitive surface, such as for example skin tissue or where eye exposure to UV cannot be avoided.

The term "mammal" refers herein to any vertebrate animal constituting the class *Mammalia* and characterized by the presence of mammary glands which in females produce milk for feeding (nursing) their young, a neocortex (a region of the brain), fur or hair, and three middle ear bones. These characteristics distinguish them from reptiles and birds, from which they diverged in the late Triassic, 201-227 million years ago. There are around 5,450 species of mammals. The largest orders are the rodents, bats and *Soricomorpha* (shrews and others). The next three are the Primates (apes, monkeys, and others), the *Cetartiodactyla* (cetaceans and even-toed ungulates), and the *Carnivora* (cats, dogs, seals, and others). This definition of mammals also includes humans.

Thus, the term "mammal skin" refers to any skin of a mammal, including the skin of livestock, wherein the term "livestock" is commonly defined as domesticated animals raised in an agricultural setting to produce labor and commodities such as meat, eggs, milk, fur, leather, and wool, such as for example cattle, goats, horses, pigs and sheep.

Furthermore, the term "mammal skin" includes also the skin of humans, such as for example patients, health care professionals, people with weak or absent immune-system (elderly, children, post-surgery, post organ transplantation, HIV-positive, etc.), people with elevated potential exposure to MRSA, etc.

Prior art UV lamp covers are made of sapphire, synthetic quartz or quartz glass (fused silica glass). However, sapphire is very expensive as compared to other transparent materials and cannot be bent, molded, drawn or melt-fused like glasses or metals. In addition, the UV-absorption at UVC wavelengths is quite high with nearly no transmission at wavelengths below 250 nm.

In an embodiment of the present disclosure, the glass has a transmission of at least 50%, better at least 60% or at least 70% at 200 nm and/or at least 85% at wavelengths [$\lambda$] of 260 nm, 280 nm and/or 310 nm (measured at a thickness of 1 mm).

Quartz and fused silica glasses, due to their high melting point, have high fabrication costs as well, since temperature and effort for melting and blowing are much higher than for other standard glasses. Furthermore, any forms other than tubes must be ground and polished from large blocks. Besides the costs of production, these covers have the disadvantage that high amounts of energy are necessary to guarantee a sufficient UV-exposure of the treated object, gas or liquid.

The glasses of the present invention, however, are suitable for forming rods, sheets, discs, tubes and bars, produced by casting, Danner, Vello and/or down-draw processes.

Furthermore, due to the high operating temperature, sagging and slumping effects may occur over time and a surface-devitrification may become visible, especially when the temperature is cycled at high temperatures. This, however, increases the weathering effects in the cover even more, which increases the UV-absorption and necessitates even higher operation energies to guarantee a sufficient UV-exposure of the treated object, gas or liquid - a vicious circle.

The high operation energy results not only in increased thermal stress to the cover, but also to the whole device, which reduces its lifetime and increases maintenance costs.

The glasses of the present invention may have excellent optical properties. In an embodiment, the glass has a refractive index $n_d$ ($\lambda$ = 587.6 nm) of 1.40 to 1.58. The refractive index can be less than 1.50.

The glasses described herein have excellent UV transmission. They may have one or more of the following optical properties:

- a UV transmission at 200 nm (measured at a thickness d = 1mm) of at least 60%, in one embodiment at least 62.5%;
- a UV transmission at 207 nm (measured at a thickness d = 1mm) of at least 65%, in one embodiment at least 67.5%;
- a UV transmission at 210 nm (measured at a thickness d = 1mm) of at least 65%, in one embodiment at least 69%;
- a UV transmission at 230 nm (measured at a thickness d = 1mm) of at least 75%, in one embodiment at least 81%; and/or
- a UV transmission at 250 nm (measured at a thickness d = 1mm) of at least 82.5%, in one embodiment at least 85.2%;

In an embodiment, the glass has a UV transmission in the wavelength region from 207 nm to 222 nm (measured at a thickness d = 1mm) ranging from at least 65% at 207 nm to at least 75% at 222 nm. In one embodiment, the UV transmission ranges from at least 67.9% at 207 nm to at least 76.8% at 222 nm.

The glass and / or the glass article preferably has a transmission of at least 50%, preferably at least 70%, at least 80%, or at least 83% at a wavelength of 254 nm. In one embodiment, the transmission at 254 nm is at most 99.9%, at most 95% or at most 90%. The transmission is measured in particular with a sample thickness of 1 mm.

For clarity: The indication that a transmission is measured at a certain wavelength does not mean that the glass is limited to the indicated thickness. Instead, the thickness indicates the thickness at which the transmission can be measured. The indication of a thickness for measurement ensures that the values can be compared. The skilled person will understand that glasses of any suitable thickness can be used in the glass covers and devices described hereinunder. In addition, the skilled person will understand that the transmission can be measured at a thickness other than 1 mm, and the transmission value at 1 mm can be calculated from such measurement.

The present invention makes use of and relates to new glasses and glass covers (lamp covers, LED cover glass) which show low UV-absorption (i.e. high UVC-transmission), thereby decreasing the operation energy and reducing the operation temperature. Furthermore, the new glasses and glass covers of this invention are comparably cheap and easy to manufacture, can be bent, molded, drawn or melt-fused to guarantee a manifold of shapes, and are resistant to most chemicals, as well as temperature and physical stress.

According to the invention, the glass has a transmission throughout the wavelength range of from 207 nm to 222 nm of at least 60% (measured at a thickness of 1 mm), wherein the glass is a borosilicate glass having a total platinum content of not more than 3.5 ppm, in some embodiments also having a low iron and titanium content of less than 5 ppm each, and a hydrolytic resistance characterized by an extracted $Na_2O$ equivalent in $\mu$g per g glass determined according to ISO 719 of not more than 250 $\mu$g/g, not more than 200 $\mu$g/g, not more than 180 $\mu$g/g, not more than 125 $\mu$g/g, not more than 50 $\mu$g/g, not more than 40 $\mu$g/g or not more than 25 $\mu$g/g.

In one embodiment it has been found that Pt-contaminations (i.e. $Pt^0$, $Pt^{2+}$, $Pt^{4+}$, and $Pt^{6+}$, also referred to as "total platinum content") in the glass may reduce the UV-transmission between 200 nm and about 250 nm. Without being bound to theory, it is assumed that platinum-contamination in the glass may induce phase-separation by the formation of nuclei within the glass. The glasses of the present invention are borosilicate glasses, with none or very low metal contamination, especially Pt-contamination of below 3.5, or below 2.5 ppm. In other embodiments between 0 and 3.5 ppm, between 0 and 2.5 ppm, between 0 and 2.0 ppm, between 0 and 1.5 ppm, between 0 and 1.0 ppm, between 0 and 0.75 ppm, between 0 and 0.5 ppm, between 0 and 0.25 are preferred. In a further embodiment the glass is free of any Pt-contamination.

In yet a further embodiment it has been found that also $TiO_2$ contamination (also referred to as "titanium content") in the glass may further reduce the UV-transmission between 200 nm and about 250 nm. Thus, in one embodiment glasses with a $TiO_2$ content of 100 ppm or less, preferably 50 ppm or less are preferred. Preferably, the amount of $TiO_2$ should be below 7 ppm, below 6 ppm, below 5 ppm, or below 4 ppm. In other embodiments the $TiO_2$ content may be between 0 and 6.9 ppm, between 0 and 5.8 ppm, between 0 and 4.7 ppm, between 0 and 3.8 ppm, or between 0 and 2.5 ppm. In optional compositions, a content of between 0 and 1.5 ppm, between 0 and 1.0 ppm, between 0 and 0.75 ppm, between 0 and 0.5 ppm, or between 0 and 0.25 ppm is preferred. In a further embodiment the glass is free of any $TiO_2$-contamination.

In yet a further embodiment it has been found that also Fe-contamination in the glass may further reduce the UV-transmission between 200 nm and about 250 nm. In this description, iron contents are expressed as parts by weight of $Fe_2O_3$ in ppm. This value can be determined in a manner familiar to the person skilled in the art by determining the amounts of all iron species present in the glass and assuming for the calculation of the mass fraction that all iron is present as $Fe_2O_3$. For example, if 1 mmol of iron is found in the glass, the mass assumed for the calculation corresponds to 159.70 mg $Fe_2O_3$. This procedure takes into account the fact that the quantities of the individual iron species in the glass cannot be determined reliably or only with great effort. In some embodiments the glass contains less than 100 ppm $Fe_2O_3$, in particular less than 50 ppm, or less than 10 ppm. In an embodiment with a particularly low iron content, the content of $Fe_2O_3$ is less than 6 ppm, less than 5 ppm, or less than 4.5 ppm. Optionally, the $Fe_2O_3$ content is between 0 and 4.4 ppm, between 0 and 4.0 ppm, between 0 and 3.5 ppm, between 0 and 2.0 ppm, or between 0 and 1.75 ppm. In some embodiments, the content can be between 0 and 1.5 ppm, or preferably between 0 and 1.25 ppm. In a further embodiment, the glass is free of any contamination with $Fe_2O_3$.

Thus, glasses have a sum of all contaminations with Pt, $TiO_2$ and $Fe_2O_3$ of below 13.5 ppm, in another embodiment below 10.5 ppm, in another embodiment below 8.5 ppm. In other embodiments glasses are preferred with a sum of all contaminations between 0 and 8.2 ppm, between 0 and 7.0 ppm, between 0 and 6.0 ppm, between 0 and 5.0 ppm, between 0 and 4.0 ppm, between 0 and 3.0 ppm, between 0 and 2.0 ppm, between 0 and 1.0 ppm, between 0 and 0.5 ppm, between 0 and 0.25 ppm. In a further embodiment the glass is free of the contaminations with at least one, two, or up to all three of the metals selected from Pt, $TiO_2$ and/or $Fe_2O_3$.

Also other contaminations with transition elements and/or heavy metals such as lead, rhodium, cadmium, mercury and hexavalent chromium may be kept below 10 ppm, in another embodiment below 8.5 ppm. In other embodiments these contaminations may be kept between 0 and 8.2 ppm, between 0 and 7.0 ppm, between 0 and 6.0 ppm, between 0 and 5.0 ppm, or between 0 and 4.0 ppm. In other embodiments, the level of these contaminations may be between 0 and 3.0 ppm, between 0 and 2.0 ppm, between 0 and 1.0 ppm, between 0 and 0.5 ppm, or between 0 and 0.25 ppm. In a further embodiment the glass is free of any transition metal and/or heavy metal-contamination.

If a reference is made herein to a chemical element, then this statement refers to any chemical form, unless otherwise stated in the individual case. For example, the statement that the glass has a content of As of less than 100 ppm means that the sum of the mass fractions of the As species present (e.g. $As_2O_3$, $As_2O_5$, etc.) does not exceed the value of 100 ppm.

As used herein, the term "ppm" means parts per million on a weight-by-weight basis (w/w). Metal contaminations during the production process need to be avoided in order to produce glasses with suitable UV-transmission. Thus, the present invention may also pertain to methods to produce glasses with high UV-transmission.

In one embodiment, the glass of the present invention is a borosilicate glass with high UV-transmission and the following additional ranges of physical and chemical parameters.

Unlike quartz, the glasses of this invention have excellent melting properties, e.g. low transformation temperatures and working points. Examples of suitable glass parameters may be selected from a transformation temperature $T_g$ (ISO 7884-8) below 550°C, such as from 400°C to 500°C, in one embodiment between 420°C and 460°C; in another embodiment between 450°C and 480°C.

The glass may have a $T_{13}$ temperature, i.e. a glass temperature at a viscosity $\eta$ in dPa*s of $10^{13}$ (annealing point) (ISO 7884-4), between 410°C and 550°C, such as in one embodiment between 445°C and 485°C; in another embodiment between 490°C and 510°C. The glass may have a softening point, i.e. the temperature at which the viscosity is $10^{7.6}$ dPa*s (softening point) (ISO 7884-3) between 650°C and 750°C, such as in one embodiment between 690°C and 715°C, in another embodiment between 700°C and 725°C. The glass may have a working point, i.e. of the temperature at which the viscosity is $10^4$ dPa*s (working point) (ISO 7884-2) between 1000°C and 1150°C, in one embodiment such as between 1060°C and 1100°C; in another embodiment between 1090°C and 1140°C. The temperature-viscosity dependence expressed by one or more of these parameters goes along with the ability of the glass to be drawn or otherwise formed into any desired shape, including UV lamp covers and UV-LED covers.

Thus, in one embodiment the glass has a $T_g$ of between 420°C and 465°C; a $T_{13}$ of between 445°C and 485°C; a softening point of between 690°C and 715°C and a working point of between 1060°C and 1100°C.

In another preferred embodiment the glass has a $T_g$ of between 460 and 470°C; a $T_{13}$ of between 490 and 510°C; a softening point of between 700 and 725°C and a working point of between 1090 and 1140°C.

The glasses of the present invention may have a density $\rho$ at 25°C between 2 and 2.5 g*cm$^{-3}$. The low density makes the glass most suitable for mobile applications, e.g. mobile MRSA eradication equipment.

The glasses of the present invention may feature a thermal conductivity $\lambda_w$ at 90°C between 0.8 and 1.2 W * m$^{-1}$ * K$^{-1}$, making it most suitable for use as lamp cover.

The UVC-glasses and the UVC-glass covers made thereof have the following additional features:
The term "solarization" refers to a phenomenon in physics where a material undergoes a change in light transmission after being subjected to high-energy electromagnetic radiation, such as ultraviolet light. Clear glass and many plastics will turn amber, green or other colors when subjected to X-radiation, and glass may turn blue after long-term solar exposure in the desert. Solarization may also permanently degrade a material's physical or mechanical properties, and is one of the mechanisms involved in the breakdown of plastics within the environment.

The glasses of the present invention may show a very good resistance against "solarization" (see example section) and, thus, are very suitable for the use as UV-glasses. "Solarization" is the reduction of transmission for light of different wavelength ranges caused by exposure to short-wave UV light. Solarization can make the glass either colored or completely opaque.

Thus, "solarization resistance" is the property of the glass to maintain a high transmission at a certain wavelength even after UV irradiation. It can be described by calculating the induced absorbance $\alpha(\lambda)$:

$$\alpha(\lambda) = -\ln \frac{T(\lambda)_i}{T(\lambda)_0}$$

with $T(\lambda)_0$ = transmission before irradiation and $T(\lambda)_i$ = transmission after *i* hours of irradiation with a deuterium lamp. The smaller $\alpha(\lambda)$ is, the more resistant the glass is to solarization. The solarization resistance is stated herein for the wavelength 200 nm. For the specification of the solarization resistance, a sample thickness of about 0.70 mm to 0.75 mm is assumed in this specification. This means that the measurement takes place at this sample thickness. The claimed glass article itself may have a different thickness. The irradiation is carried out with a deuterium lamp. Deuterium lamps emit light up to a very short-wave UV range. The lamp used here has a cut-off wavelength of 115 nm. The power of the deuterium lamp can be about 1 W/m$^2$. The following deuterium lamp (DUV) can be used: Heraeus Noblelight GmbH, Type V04, S-Nr.: V0390 30 W, with MgF$_2$ filter for sufficient emission up to 115 nm.

The glasses used in this invention also show a very good hydrolytic resistance and high gas-tightness.

The phase separation factor is a measure of the property of the glass to change its hydrolytic resistance as defined in ISO 719 as a result of phase separation. Phase separation occurs when the glasses are fused due to the influence of temperature. It has been proven to be advantageous to select glasses with a phase separation factor as close to 1 as possible, so that the glass properties of a phase separated glass do not differ greatly from the raw glass in terms of its hydrolytic resistance. The phase separation factor is influenced by the composition of the glass, but also by its thermal history (cooling state).

The phase separation factor *E* is calculated as follows.

$$E - Faktor = \frac{Equ_{roh}}{Equ_{ent}}$$

Therein, $Equ_{roh}$ and $Equ_{ent}$ are the extracted Na$_2$O equivalents in µg per g glass determined according to ISO 719:1989-12 of the non-phase separated and phase separated glass, respectively. The phase separation factor is a property of the glass. This factor does not mean that the claimed glass has undergone phase separation, but that, if phase separation happens, the influence on hydrolytic stability is in the range given by the factor. Every glass can be analyzed for its phase separation factor. For that purpose, the extracted Na$_2$O equivalents are determined in a phase separated specimen and a non-phase separated specimen. For the purpose of measurement, a "phase separated glass" is obtained by holding a glass specimen at 100°C above the glass transition temperature ($T_g$) for 4 hours. This temperature treatment ensures a certain level of phase separation.

The hydrolytic resistance may be expressed as the extracted Na$_2$O equivalent in µg per g glass. The extracted Na$_2$O equivalent in µg per g glass is determined according to ISO 719:1989-12. It is a measure of the extractability of basic compounds from glass in water at 98 °C.

In one embodiment, the glass has a phase separation factor with regard to its hydrolytic resistance in the range of between 0.1 and 1.65, or between 0.2 and 1.65, or between 0.35 and 1.65, or between 0.40 and 1.65, or between 0.65 and 1.65, in particular between 0.70 and 1.10. In particular, the factor is at least 0.1, or at least 0.2, or at least 0.35, or at least 0.40 or at least 0.70. Preferably, this factor is close to 1.00, which corresponds to the case of unchanged hydrolytic resistance after phase separation. In one version, the phase separation factor is up to 1.40, up to 1.25 or up to 1.10.

In one embodiment the factor is at least 0.70 and up to 1.6. In another version, the phase separation factor is at least 0.30 and up to 0.5.

Due to the glass properties, the UVC-glass covers can be sealed hermetically, for example by the use of laser glass frit sealing. This hermetical sealing is important, since a number of UVGI-applications take place in either aqueous environments (e.g. biofilm treatment or water treatment), humid environments (e.g. sewage systems) and/or environments with elevated gas-pressure or under vacuum. Furthermore, the hermetical sealing allows the final device, e.g. a UVC-LED-lamp, to be autoclaved, so that it can be used in hospitals, in surgery, in laboratories or in any other environment where high hygienic standards are needed.

This is contrary to conventional glasses, quartz and/or fused silica glasses, which do not possess the thermic properties needed for laser frit sealing, and, thus, cannot be sealed hermetically. However, the glasses described herein are suitable to achieve crack free and tight glass frit connections.

The glasses of this invention preferably have a product CTE $[°C^{-1}]$ x $T_4$ $[°C]$ of at most 0.0055, more preferably at most 0.0053 or at most 0.0051. The product may be at least 0.0044 or at least 0.0045. It has been shown that these glasses show advantageous properties with regard to fusion stress and melting behavior.

"$T_4$" is the temperature at which the glass has a viscosity of $10^4$ dPa*s. $T_4$ can be measured by the methods known to the person skilled in the art for determining the viscosity of glass, e.g. according to DIN ISO 7884-1: 1998-02. "$T_{13}$" is the temperature at which the glass has a viscosity of $10^{13}$ dPa*s.

The average linear coefficient $\alpha$ of the thermal expansion (CTE) (at 20°C; 300°C, according to ISO 7991) is in one embodiment between 3.0 and 6.0 * $10^{-6}$ $K^{-1}$. The thermal expansion coefficient (CTE) may be less than 4.5 * $10^{-6}$ $K^{-1}$. It may range from 3.5 to < 5 * $10^{-6}$ $K^{-1}$, more preferably from 3.75 to 4.75 * $10^{-6}$ $K^{-1}$, more preferably from 4.1 to 4.6 * $10^{-6}$ $K^{-1}$, still more preferably from 4.1 to 4.5 * $10^{-6}$ $K^{-1}$. This allows adapting the thermal expansion properties to the overall thermal expansion properties of the UV-device and therefore prevents tensions within the glass cover. In one embodiment, the same or similar CTE is chosen for both the UVC-glass cover as well as the underlying UV-device (e.g. UVC-LED-package).

Preferably, the glass transition temperature is below 500°C. It may be in a range of from 400°C to 550°C, more preferably between 410°C and 500°C, in another embodiment in a range of between 420°C and 480°C. The processing temperature $T_4$ is the temperature at which the glass viscosity is $10^4$ dPa*s. The processing temperature $T_4$ of the glasses of the present invention may be below 1200°C, in some embodiments below 1125°C. It may be in a range of between 1000°C and 1200°C, more preferably in a range of between 1025°C and 1175°C.

In order that the melting properties, including $T_g$ and $T_4$, are in the desired range, it may be advantageous to set the ratio of the content $B_2O_3$ to the sum of $SiO_2$ and $Al_2O_3$ (in mol%) in a narrow range. In an advantageous embodiment, this ratio is at least 0.15 and/or at most 0.4.

Another important property of the glasses is their excellent spatial homogeneity of the refractive index $n_d$ of the material. Optionally, a refractive index variation within the glass may correspond to a deformation of the wavefront passing through the glass, according to the following formula:

$$\Delta s = \Delta(n_d {*} d) = \Delta n_d {*} d + \Delta d {*} n_d$$

wherein $\Delta s$ is the wavefront deviation, d is the thickness of the glass, $\Delta d$ is the thickness variation (difference between maximum and minimum thickness) and $\Delta n_d$ is the refractive index variation (difference between maximum and minimum refractive index) in the glass. The invention further includes glass articles having the indicated wavefront deviation.

The wavefront deviation can be calculated according to the formula above. The refractive index $n_d$ ($\lambda$ = 587.6 nm) and the thickness may be determined at 20°C. In one embodiment the wavefront deviation is determined over and/or applies to a surface area of 1 $cm^2$. The wavefront deviation may be determined for a thickness of 10 mm of glass, or less; or 1 mm of glass or less. Optionally, the thickness may be at least 200 $\mu$m. The wavefront deviation may be less than $\pm$ 0.1 mm, less than $\pm$ 0.08 mm, in further embodiments less than $\pm$ 0.035 mm, less than $\pm$ 25 $\mu$m, less than $\pm$ 15 $\mu$m, or less than $\pm$ 5 $\mu$m. Optionally, the wavefront deviation may be between 0.1 $\mu$m and 250 $\mu$m, or between 1 $\mu$m and 100 $\mu$m, or between 2 $\mu$m and 85 $\mu$m.

The wavefront deviation may be measured axial, e.g. in the case of glass tubes, as used for example in discharge lamps; or lateral, e.g. in the case of rod sections, as used for lenses in UVC-LEDs.

The wavefront may also be measured by a wavefront sensor. This is a device which measures the wavefront aberration in a coherent signal to describe the optical quality or lack thereof in an optical system. Without being bound to a specific method, a very common method is to use a Shack-Hartmann lenslet array.

Alternative wavefront sensing techniques to the Shack-Hartmann system are mathematical techniques like phase imaging or curvature sensing. These algorithms compute wavefront images from conventional brightfield images at different focal planes without the need for specialized wavefront optics.

The glasses and glass articles according to the present invention may have a low content of wavefront deformations in the glass (striae, bubbles, streaks, etc.). In general, it can be distinguished between the global or long-range homogeneity of refractive index in the material and short-range deviations from glass homogeneity. Striae are spatially short-range variations of the homogeneity in a glass. Short-range variations are variations over a distance of about 0.1 mm and up to 2 mm, whereas the spatially long range global homogeneity of refractive index covers the complete glass piece.

In some embodiments an ultraviolet ray transmission filter may be used, which filters out certain non-desired UV-wavelengths, e.g. wavelengths below 207 nm, in some embodiments below 200 nm; and/or above 222 nm, in some embodiments above 250 nm.

The glasses for UV-covers according to the present invention may allow the shaping of lenses in order to optically shape the UV-beam, for example for directional focusing of the UV-light to the target.

Any beam angle between 10° and 180° is possible. In some embodiments 10° to 20°, 20° to 30°, 30° to 40°, 40° to 50°, 50° to 60°, 60° to 70°, 70° to 80°, 80 to 90° may be used. In other embodiments 15° to 35°, 25° to 45°, 35° to 60°, 45° to 90°, 75° to 120°, 90° to 145°, 120° to 180° may be used.

In some embodiments rather broad beam shapes, such as 90°, 120° or even 180°, are useful, for example in cases in which a surface of a certain size or a certain volume or a tube of a certain diameter needs to be decontaminated in one treatment.

In other embodiments narrow beam shapes such as 10°, 5° or even 1°, are useful. For example narrow beam shapes can be used to concentrate the UV-exposure at the target site and avoid non-directional and undesired radiation, which would result in a less efficient energy-to-radiation ratio or exposure of UV-sensitive surfaces to UV-light. One example may be the limited decontamination of defined areas of an eye.

Furthermore, different lens shapes and beam angles may be used to solve complex MRSA-eradication tasks. For example in cases in which UV-sensitive surfaces of different sensitive levels are next to each other and need to be UV-exposed in one treatment.

For example, it may be suitable to treat the skin of a patient in some areas with higher UV-exposure than in neighboring areas, e.g. during wound treatment and/or surgery, where the wound itself is exposed to less UV than the surrounding skin.

The discolure includes also methods for the production of LED-packages with covers made with the inventive UVC-transparent glasses.

An LED package according to the present disclosure may comprise

- an LED chip,
- optionally: a substrate whereon the LED chip is mounted - e.g. made of PCB, polymer, inorganic material esp. ceramic, metal;
- optionally: a base plate in case including feedthroughs for electrical conductors for contacting the LED chip (metals, ceramics, glass ceramics, seldom polymers);
- a frame containing the LED chip being attached to or surrounding the base plate and building some kind of a cavity (metals, ceramics, glass ceramics, seldom polymers);
- a distal portion of the housing (cover) facing away and in distance from the chip being at least partially transparent closing the package or being made in total of a transparent material; wherein at least the transparent part of the cover is made of the UVC-transparent glass described herein.

Such windows may be flat or having a shape for changing the path of the light (i.e. lens-shape).

As mentioned before, UVC-LEDs may be packaged and sealed in a way (e.g. laser frit sealing) so that they are autoclavable, sterilizable and resistent against fluids. Such UVC-LEDs may be used for sterilization of air and water, surfaces; and for medical/dental applications.

The UVC-LEDs having the UVC-transparent glass described herein possess further advantages in comparison to conventional UVGI-lamps or devices (such as mercury-vapor lamps), for example:

- Instant on/off-function which allows an "on-demand disinfection" without wasted energy;
- Directional emission (especially by the use of lenses which allow the control of the beam angle), which allows "targeted disinfection" with simple design;
- Semiconductor durability, which allows the use in rugged, portable devices;
- Low DC-power requirement, which increases the energy-efficacy and results in simple, inexpensive electrical drivers;
- Compact packaging, which maximizes the design flexibility;
- Eco-friendly construction, since it allows easy disposal without harmful mercury exposure;
- High optical performance;
- High radiation power at defined wavelength;
- CTE matching with AIN;
- Comparably low production price;
- Small size

Traditionally, both low and medium pressure mercury lamps have been utilized in disinfection systems. However, there is a need to replace these light sources with high powered and energy-efficient UV-lights, such as for example UVC-LEDs. The UV-lamps and the UV-devices under this invention are more energy efficient as compared to conventional UVGI-lamps or - devices. This is because the inventive glass may transmit more than 60% of the UV-light at 200 nm and therefore the ratio between energy-input and radiation-output is significantly improved.

This becomes important when these glasses are used as covers of UVC-LED-lamps. If the energy requirement of a conventional mercury-vapor-UV-lamp is set at 100%, the energy necessary to generate the same UV-radiation with the UVC-LEDs described herein is about 10 to 30%. In other words, if a conventional UV-lamp uses 10 W of energy to emit a certain UV-intensity the devices herein may use only between 1 and 3 W.

As mentioned before, another advantage of the UVC-transparent glasses described herein are their high thermal conductivity ($\lambda_w$), which may be between 0.75 and 1.25 W * m$^{-1}$ * K$^{-1}$ at 90°C, in other embodiments about 1.0 W * m$^{-1}$ * K$^{-1}$. This superior thermal conductivity increases the lifetime of the device, since excess heat can dissipate easily before harming other parts of the device. This is for example in contrast to quartz-glasses, which normally have a less optimal thermal conductivity.

Thus, in one embodiment, the method according to present invention may include UV-lamps with an energy efficiency index (EEI) of $\leq 0.11$ according to the REGULATION (EU) No 874/2012 in case of non-directional UV-lamps and an energy efficiency index (EEI) of $\leq 0.13$ according to the REGULATION (EU) No 874/2012 in case of directional UV-lamps.

The methods described herein may be used for eradication of MRSA from any kind of surface, including UV-sensitive surfaces, UV-sensitive liquid and/or UV-sensitive gas.

Of course the methods described herein may be used also for eradication other UV-sensitive pathogenic organisms, such as viruses (such as for example influenza- or coronaviridae, such as SARS-CoV-2, especially resistant virus mutations, such as for example SARS-CoV2-D614G), bacteria (including spores), pathological yeasts, mold, and the like.

Potential applications can be selected from the list of uses comprising hand sanitizers (e.g. on private and public toilets), room sanitizers in health care environments, MRSA-eradication in preparation to or during or after surgery, wound treatment, eye treatment, food disinfection (e.g. during food production and/or meat, dairy or vegetable counter in supermarkets), livestock disinfection (especially in cases of intensive animal husbandry, such as for example laying batteries), production of pharmaceutical compounds and/or food production processes, storage facilities and/or disinfection of UV-sensitive surfaces which are often in contact with many different users, for example keyboards, handles, handrails, tooth brushes, hair brushes, ornaments, touch-devices, shaving razors, or kids toys.

The UVC-devices disclosed herein can also be used for a wide range of applications as "analytical instrumentation", for example:

- HPLC (high-performance liquid chromatography): Used in analysis for detecting chemicals and compounds in life sciences;
- Spectrometers: Used in multiple applications in tests and analysis across biotech, life sciences and environmental monitoring; and
- Water quality monitoring sensors: Used for detecting chemicals in water (e.g. in the course of fracking, in cases of general water security or before the disposal of treated wastewater).

In another embodiment the UVC-devices disclosed herein may comprise devices for "water disinfection". In that respect UVC LEDs are advantageous as compared to traditional mercury lamps, which require a long warm up time (anywhere from 50 seconds to 10 minutes) to reach the required germicidal intensity. In addition, frequent on/off cycles can diminish lifetime by 50 percent or more.

Consequently, mercury lamps in these applications need to be kept on all day, increasing the frequency of lamp replacement and rising power consumption. By contrast, the instant on-off capability of UVC LEDs enables on-demand disinfection, which reduces power consumption significantly.

Additionally, the frequent on/off-cycles do not diminish LED lifetime, helping lower operating and maintenance costs.

Further uses of the UVC-devices disclosed herein especially in cases of UV-sensitive surfaces, liquids or gases, may comprise:

- Protein analysis, i.e. the bioinformatics study of protein structure and function using database searches, sequence comparisons, structural and functional predictions.
- Molecular identification, i.e. a process of comparing specific pieces of DNA between organisms.
- Cytometry, i.e. in biotechnology, flow cytometry is a laser- or impedance-based, biophysical technology employed in cell counting, cell sorting, biomarker detection and protein engineering, by suspending cells in a stream of fluid and passing them through an electronic detection apparatus.
- Biofilm treatment systems, i.e. systems which employ the use of bacteria, fungi, algae, and protozoa to remove organic and inorganic materials from the surrounding liquid.
- Nitrate and/or NOx-measurement, which is normally done at wavelengths of about 230 nm.
- Paracetamol-concentration-measurement, which is normally done at wavelengths of about 245 nm; and/or
- Skin treatment in order to improve dermatologic conditions (e.g. psoriasis, vitiligo, itching, neurodermatitis, acne, actinic dermatitis, phototherapy, pityriasis rosea, etc.).

Thus, in one embodiment the use of the UV-LED-module of the present disclosure may be selected from the group of water disinfection, analytical instrumentation (HPLC, spectrometers, water monitoring sensors), air purification, air disinfection, surface disinfection (e.g. keyboard disinfection, escalator handrail UV sterilizer), cytometry, molecular identification, protein analysis, biofilm treatment, curing, lithography, vegetable growth, skin cure, germ detection, drug discovery, protein analysis, induction of skin vitamine-D3-production and/or sterilization. Thus, in one aspect the invention pertains to uses of the glass according to the invention as a hermetically sealing lens cap for an UV-LED-module, e.g. for applications selected from the group of water disinfection, analytical instrumentation (HPLC, spectrometers, water monitoring sensors), air purification, air disinfection, surface disinfection (e.g. keyboard disinfection, escalator handrail UV sterilizer), cytometry, molecular identification, protein analysis, biofilm treatment, curing, lithography, vegetable growth, skin cure (psoriasis, vitiligo, itching, neurodermatitis, acne, actinic dermatitis, phototherapy, pityriasis rosea,), germ detection, drug discovery, protein analysis, induction of skin vitamine-D3-production and/or sterilization.

The glass is a borosilicate glass.

According to the disclosure borosilicate glass comprises the following components (in mol% based on oxides):

| Component | Content [mol%] |
|---|---|
| $SiO_2$ | 40 to 85 |
| $Al_2O_3$ | 0 to 25 |
| $Na_2O$ | 0 to 18 |
| $K_2O$ | 0 to 15 |
| MgO | 0 to 10 |
| $B_2O_3$ | 5 to 24 |
| $Li_2O$ | 0 to 10 |
| ZnO | 0 to 5 |
| CaO | 0 to 16 |
| BaO | 0 to 12 |
| $ZrO_2$ | 0 to 5 |
| $SnO_2$ | 0 to 3 |
| SrO | 0 to 4 |
| $F^-$ | 0 to 6 |
| $Cl^-$ | 0 to 1 |

According to the invention borosilicate glass comprises the following components (in mol% based on oxides):

| Component | Content [mol%] |
|---|---|
| $SiO_2$ | 60 to 78 |
| $Al_2O_3$ | 0 to 10 |
| $B_2O_3$ | 12 to 24 |
| $Li_2O$ | 0 to 3 |
| $Na_2O$ | 0 to 6 |
| $K_2O$ | 0 to 4 |
| MgO | 0 to 6 |

(continued)

| Component | Content [mol%] |
|-----------|----------------|
| CaO | 0 to 6 |
| SrO | 0 to 4 |
| BaO | 0 to 4 |
| F⁻ | 0 to 6 |
| Cl⁻ | 0 to 0.5 |
| $R_2O$ | 3.5 to 10 |
| RO | 0 to 6 |

Wherein "$R_2O$" refers to the alkali metal oxides $Li_2O$, $Na_2O$ and $K_2O$; and "RO" denotes the alkaline earth metal oxides MgO, CaO, BaO and SrO.

The glasses of the present invention may contain $SiO_2$ in a proportion of at least 40 mol%, or at least 60 mol%. $SiO_2$ contributes to the hydrolytic resistance and transparency of the glass. If the $SiO_2$ content is too high, the melting point of the glass is too high. The temperatures $T_4$ and $T_g$ also rise sharply. Therefore, the content of $SiO_2$ should be limited to a maximum of 78 mol%, or to a maximum of 85%.

Preferably the content of $SiO_2$ is at least 61 mol%, at least 63 mol% or at least 65 mol%, at least 68 mol%, at least 69 mol%, or at least 70 mol%. The content can be limited to a maximum of 75 mol% or a maximum of 73 mol%, or a maximum of 72 mol%.

The glasses of the present invention contain $Al_2O_3$ in a maximum proportion of 10 mol%. $Al_2O_3$ contributes to the phase separation stability of the glasses, but in larger proportions reduces the acid resistance. Furthermore, $Al_2O_3$ increases the melting temperature and $T_4$. Thus, the content of this component should be limited to a maximum of 25 mol%, or to a maximum of 9 mol%, or to a maximum of 8 mol%, or to a maximum of 7 mol%, or to a maximum of 5 mol%, or to a maximum of 4.5 mol%. In some embodiments $Al_2O_3$ is used in a small proportion of at least 2 mol%, at least 2.5 mol%, or at least 3 mol%, or at least 3.25 mol%. In some embodiments the glass may be free of $Al_2O_3$.

The glasses of the present invention may contain $B_2O_3$ in a proportion of at least 12 mol%. $B_2O_3$ has a beneficial effect on the melting properties of glass, in particular, the melting temperature is lowered and the glass can be fused with other materials at lower temperatures.

However, the amount of $B_2O_3$ should not be too high, otherwise the glasses have a strong tendency to phase separation. In addition, too much $B_2O_3$ has a negative effect on the hydrolytic resistance and the glass tends to have a high evaporation loss during production, resulting in a glass with knots. Thus, $B_2O_3$ should be limited to up to 24 mol%, up to 22 mol%, or up to 20 mol%. The content of $B_2O_3$ can be at least 5 mol%, at least 12 mol%, or at least 14 mol%.

In a preferred design, the ratio of the sum of the contents (in mol%) of $B_2O_3$, $R_2O$ and RO to the sum of the contents (in mol%) of $SiO_2$ and $Al_2O_3$ is at most 0.4, in particular at most 0.35, more preferably at most 0.34. In one embodiment, this value is at least 0.1, preferably at least 0.2, or at least 0.26. Glasses with the above-mentioned proportion have good properties in terms of hydrolytic resistance and phase separation factor, and they have only a low induced extinction, which has many advantages, especially when used as UV-transparent material.

The glasses of the present invention may contain $Li_2O$ in a proportion of up to 10.0 mol%, or up to 3.0 mol%, or up to 2.8 mol%, or up to 2.5 mol%. $Li_2O$ increases the fusibility of the glasses and results in a beneficial shift of the UV edge to lower wavelengths. However, lithium oxide tends to evaporate, increases the tendency to phase separation and also increases the price of the mixture. In the preferred design, the glass contains only a small amount of $Li_2O$, e.g. to a maximum of 3.0 mol%, to a maximum of 2.8 mol%, to a maximum of 2.5 mol%, to a maximum of 2.0 mol%, or to a maximum of 1.9 mol%, or the glass is free of $Li_2O$. In certain embodiments the content of $Li_2O$ is between 1 mol% and 2 mol%.

The glasses of the invention contain $Na_2O$ in a proportion of up to 18 mol%, or up to 6 mol%. $Na_2O$ increases the fusibility of the glasses. However, sodium oxide also leads to a reduction in UV transmission and an increase in the coefficient of thermal expansion (CTE). The glass may contain $Na_2O$ in a proportion of at least 1 mol%, or at least 2 mol%. In one version the content of $Na_2O$ is a maximum of 5 mol%, or a maximum of 4 mol%. In some embodiments the glass may be free of $Na_2O$.

The glasses of the present invention contain $K_2O$ in a maximum proportion of 4 mol%. $K_2O$ increases the fusibility of the glasses and results in a beneficial shift of the UV edge to lower wavelengths. Its content may be at least 0.3 mol%, or at least 0.75 mol%. However, a potassium oxide content that is too high leads to a glass that has a disturbing effect when used in photomultipliers due to the radiating property of its isotope [40]K. Therefore, the content of this component must be limited to a maximum of 15 mol%, to a maximum of 10 mol%, to a maximum of 5 mol%, to a maximum of 3 mol%, or a maximum of 2 mol%. In some embodiments the glass may be free of $K_2O$.

In an embodiment of the invention, the ratio of the contents of $Na_2O$ to $K_2O$ in mol% is at least 1.5, in particular at least 2. In an embodiment of the invention the said ratio is at most 4, in particular at most 3. Both oxides serve to improve the fusibility of the glass. However, if too much $Na_2O$ is used, the UV transmission is reduced. Too much $K_2O$ increases the coefficient of thermal expansion. It was found that the ratio given achieves the best results, i.e. the UV transmission and the coefficient of thermal expansion are in advantageous ranges. In certain embodiments the ratio is between 1.85 and 3.

The amount of $R_2O$ in the glasses of the present invention is preferably not more than 10 mol%, not more than 8 mol%, or not more than 7 mol%. The glasses may contain $R_2O$ in amounts of at least 3.5 mol%, at least 4 mol%, or at least 4.5 mol%. Alkali metal oxides increase the fusibility of the glasses, but, as described above, lead to various disadvantages in higher proportions. In certain embodiments the content of $R_2O$ is between 4.5 mol% and 6.0 mol%.

The glasses of the present invention may contain MgO in a proportion of up to 10 mol%, up to 6 mol%, up to 4 mol%, or up to 2 mol%. MgO is advantageous for fusibility, but in high proportions it proves to be problematic with regard to the desired UV transmission and the tendency to phase separation. Preferred designs are free of MgO.

The glasses of the present invention may contain CaO in a proportion of up to 16 mol%, up to 6 mol%, up to 4 mol%, or up to 2 mol%. CaO is advantageous for fusibility, but in high proportions it proves to be problematic with regard to the desired UV transmission. Preferred forms are free of CaO or contain only little CaO, e.g. at least 0.1 mol%, at least 0.3 mol%, or at least 0.5 mol%.

The glasses of the present invention may contain SrO in a proportion of up to 4 mol%, up to 1 mol%, or up to 0.5 mol%. SrO is advantageous for fusibility, but in high proportions it proves to be problematic with regard to the desired UV transmission. Preferred designs are free from SrO.

The glasses of the present invention may contain BaO in a proportion of up to 12 mol%, or up to 4 mol%, or up to 2 mol%. BaO leads to an improvement of the hydrolytic resistance. However, a too high barium oxide content leads to phase separation and, thus, to instability of the glass. Preferred embodiments contain BaO in amounts of at least 0.1 mol%, at least 0.3 mol%, or at least 0.4 mol%. In certain embodiments the content of BaO is between 0.3 mol% and 1.5 mol%. In some embodiments the glass may be free of BaO.

It has been shown that the alkaline earth oxides RO have a great influence on the phase separation tendency. In a design form, special attention is therefore paid to the contents of these components and their relationship to one another. Thus, the ratio of BaO in mol% to the sum of the contents of MgO, SrO and CaO in mol% should be at least 0.4. Preferably, this value is at least 0.55, or at least 0.7, or at least 1.0. In particularly preferred forms, the value is at least 1.5, or even at least 2. BaO offers the most advantages in terms of phase separation and hydrolytic resistance compared to the other alkaline earth metal oxides. However, the ratio should not exceed 4.0 or 3.0. In advantageous forms, the glass contains at least small amounts of CaO and BaO and is free of MgO and SrO. In certain embodiments the ratio is between 0.7 and 2.2.

Advantageous properties are obtained in particular if the ratio of the proportion of CaO in the glass to BaO in mol% is less than 2.0. In particular, this ratio should be less than 1.5 or less than 1.0. In some embodiments the ratios are even lower, in particular less than 0.8, or less than 0.6, and in a preferred design this ratio is at least 0.3. In certain embodiments the ratio is between 0.4 and 1.4.

In one version, the glass has a mol% ratio of $B_2O_3$ to BaO of at least 8 and at most 45. Preferably, the ratio is at least 10, or at least 11 and, in a preferred design, the said ratio is limited to a maximum of 42, or of 40, or of 39. In another embodiment, the ratio may be limited to a maximum of 15 or 14. In particular, the ratio is not less than 10 and not more than 45, or in another embodiment not less than 11 and not more than 42; in certain embodiments the ratio is between 11 and 16. In one other embodiment the ratio is between 35 and 45. Glasses with the above ratios show good properties in terms of hydrolytic resistance and phase separation factor, as well as low induced absorbance.

The proportion of RO in the glasses of the present invention can be at least 0.3 mol%. Alkaline earth metal oxides are advantageous for fusibility, but in high proportions they prove to be problematic with regard to the desired UV transmission. In one version, the glass contains a maximum of 3 mol% RO. In one embodiment the proportion of RO is between 1 and 3 mol%.

The sum of the contents in mol% of alkaline earth metal oxides and alkali metal oxides, $RO+R_2O$, can be limited to a maximum of 10 mol%. Advantageous designs can contain these components in quantities of maximum 9 mol%. Preferably the content of these oxides is at least 4 mol%, at least 5 mol%, or at least 6 mol%. In one embodiment the $RO+R_2O$-proportion is between 6 and 8 mol%. These components increase the phase separation tendency and reduce the hydrolytic resistance of the glasses in too high proportions.

The ratio of the contents in mol% of $B_2O_3$ to the sum of the contents of $R_2O$ and RO in mol% may be at least 1.3, at least 1.5, or at least 1.8. The ratio can be limited to a maximum of 6, a maximum of 4.5, or a maximum of 3. In one embodiment the $B_2O_3/(RO+R_2O)$-proportion is between 1.8 and 3.5. Alkali or alkaline earth borates can form during glass phase separation, if too much alkali or alkaline earth oxide is present in relation to $B_2O_3$. It has been proven to be advantageous to adjust the above ratio.

To ensure that the melting properties, including $T_g$ and $T_4$, are within the desired range, it may be advantageous to set the ratio of the content of $B_2O_3$ to the sum of the contents of $SiO_2$ and $Al_2O_3$ in mol% within a narrow range. In an advantageous design, this ratio is at least 0.15 and/or at most 0.4. In one embodiment the $B_2O_3/(SiO_2 + Al_2O_3)$-ratio is

between 0.17 and 0.3.

The ratio of the proportions in mol% of the sum of the alkali metal oxides $R_2O$ to the sum of the alkaline earth metal oxides RO is preferably >1, in particular >1.1 or >2. In the design forms, this ratio is at most 10, at most 7 or at most 5. In one embodiment the ratio is between 2 and 4.

The glasses of the present invention may contain $F^-$ in a content of 0 to 6 mol%. Preferably the content of $F^-$ is at most 4 mol%. In a design form, at least 1 mol%, or at least 2 mol% of this component is used. Component $F^-$ improves the fusibility of the glass and influences the UV edge towards smaller wavelengths.

The glasses of the present invention may contain $Cl^-$ in a content of less than 1 mol%, especially less than 0.5 mol%, or less than 0.3 mol%. Suitable lower limits are 0.01 mol%, or 0.05 mol%.

The glasses of the present invention may contain ZnO in a content of less than 5 mol%, especially less than 2.5 mol%, or less than 1 mol%. Suitable lower limits are 0.01 mol%, or 0.05 mol%. In some embodiments the glass may be free of ZnO.

The glasses of the present invention may contain $ZrO_2$ in a content of less than 5 mol%, less than 2.5 mol%, or especially less than 1 mol%. Suitable lower limits are 0.01 mol%, or 0.05 mol%. In some embodiments the glass may be free of $ZrO_2$.

The glasses of the present invention may contain $SnO_2$ in a content of less than 3 mol%, especially less than 2 mol%, or less than 1 mol%. Suitable lower limits are 0.01 mol%, or 0.05 mol%. In some embodiments the glass may be free of $SnO_2$.

When this description states that the glass is free of a component or does not contain a certain component, it means that this component may at most be present as an impurity. This means that it is not added in significant quantities. Non-significant quantities are quantities of less than 0.5 ppm, preferably less than 0.25 ppm, preferably less than 0.125 ppm and most preferably less than 0.05 ppm.

In one embodiment, the glass has less than 10 ppm $Fe_2O_3$, in particular less than 5 ppm or less than 1 ppm. In one embodiment, the glass has less than 10 ppm $TiO_2$, in particular less than 5 ppm or less than 1 ppm. In one embodiment, the glass has less than 3.5 ppm arsenic, in particular less than 2.5 ppm or less than 1.0 ppm. Preference is given to glass containing less than 3.5 ppm antimony, less than 2.5 ppm antimony, or less than 1.0 ppm antimony. Besides the negative effects on UV-transmission and solarization, especially arsenic and antimony are toxic and dangerous to the environment and should be avoided.

In a particularly preferred design, borosilicate glass includes the following components (in mol% on oxide basis):

| Component | Content [mol%] |
|-----------|----------------|
| $SiO_2$ | 68 to 75 |
| $Al_2O_3$ | 2 to 7 |
| $B_2O_3$ | 12 to 24 |
| $Li_2O$ | 0 to 3.0 |
| $Na_2O$ | 1 to 5 |
| $K_2O$ | 0 to 3 |
| CaO | >0 to 4 |
| SrO | 0 to 1 |
| BaO | 0 to 4 |
| $F^-$ | 0 to 6 |

In another particularly preferred form, the glass includes the following components in mol%:

| Component | Content [mol%] |
|-----------|----------------|
| $SiO_2$ | 69 to 73 |
| $Al_2O_3$ | 2.5 to 5 |
| $B_2O_3$ | 14 to 22 |
| $Li_2O$ | 1 to 2.5 |
| $Na_2O$ | 1 to 4 |

(continued)

| Component | Content [mol%] |
|-----------|----------------|
| $K_2O$ | 0.3 to 2 |
| CaO | 0.3 to 2 |
| SrO | 0 to 0.5 |
| BaO | 0.1 to 2 |
| $F^-$ | 1 to 4 |

In yet another particularly preferred form, the glass includes the following components in mol%:

| Component | Content [mol%] |
|-----------|----------------|
| $SiO_2$ | 70 to 72 |
| $Al_2O_3$ | 3.25 to 4.5 |
| $B_2O_3$ | 14 to 20 |
| $Li_2O$ | 1 to 2 |
| $Na_2O$ | 2 to 4 |
| $K_2O$ | 0.75 to 2 |
| CaO | 0.45 to 2 |
| SrO | 0 to 0.5 |
| BaO | 0.3 to 1.5 |
| $F^-$ | 2 to 4 |

The glass article can be produced by drawing processes known for glass tubes and rods. Depending on the desired shape, the person skilled in the art will choose a suitable manufacturing process, e.g. ingot casting for bars, floating or down draw for producing panes. Preferably, the cooling of the glass in the process is adjusted so that the desired properties are achieved.

In one embodiment, the glass article is produced using the Danner or the Vello method. In the Vello method, the glass melt flows vertically downwards (in the direction of the gravitational force) through a shaping tool made of an outlet ring and a needle. The shaping tool forms a negative form (matrix) of the generated cross-section of the glass tube or the glass rod. In the manufacture of glass tubes, a needle is arranged as a shaping part in the center of the shaping tool.

The difference between the Vello and the down draw method is first of all that the glass melt in the Vello method is deflected horizontally after it leaves the forming tool and, secondly, in the fact that the needle has a passage in the Vello method, through which blown air flows. As with the Danner method, the blown air ensures that the resulting glass tube does not collapse. In the down draw method, the solidified glass melt is separated without prior redirection. Since there is no redirection, one can also refrain from the use of blown air during the production of glass tubes.

In an embodiment, the disclosure relates to a glass article made of the glass disclosed herein.

The thickness of the glass article, in particular the wall thickness in the case of a glass tube, can be at least 0.1 mm or at least 0.3 mm. The thickness can be limited to up to 3 mm or up to 2 mm. The outside diameter of the glass article, e.g. the outside diameter of a glass tube or glass rod, can be up to 50 mm, up to 40 mm, or up to 30 mm. The outside diameter can in particular be at least 1 mm, at least 2 mm, or at least 3 mm. In one embodiment, the article has a thickness that is at least 3 mm and/or at most 20 mm. Optionally, the thickness is at least 5 mm, at least 6 mm, or at least 8 mm. The thickness may be limited to a maximum of 20 mm, up to 16 mm, up to 14 mm, or up to 12 mm. In one embodiment, the article has a length and a width, in particular the length being greater than the width. The length may be at least 20 mm, at least 40 mm or at least 60 mm. Optionally, it is at most 1000 mm, at most 600 mm, at most 250 mm or at most 120 mm. Preferably, the length is from 20 mm to 1000 mm, from 40 mm to 600 mm, or from 60 mm to 250 mm. The width may be at least 10 mm, at least 25 mm, or at least 35 mm. Optionally, the width is at most 575 mm, at most 225 mm or at most 110 mm. Preferably, the width is from 10 mm to 575 mm, from 25 mm to 225 mm, or from 35 mm to 110 mm.

**Thermal and/or chemical tempering**

Optionally, the manufacturing process comprises the step of chemical and/or thermal tempering of the glass article. The "tempering" is also referred to as "hardening" or "toughening". Preferably, the glass article is toughened on at least one surface, in particular thermally and/or chemically toughened. For example, it is possible to chemically temper glass articles by ion exchange. In this process, small alkali ions in the article are usually replaced by larger alkali ions. Often, the smaller sodium is replaced by potassium. However, it is also possible that the very small lithium is replaced by sodium and/or potassium. Optionally, it is possible that alkali ions are replaced by silver ions. Another possibility is that alkaline earth ions are exchanged for each other according to the same principle as the alkali ions. Preferably, the ion exchange takes place in a bath of molten salt between the article surface and the salt bath. Pure molten salt, for example molten $KNO_3$, can be used for the exchange. However, salt mixtures or mixtures of salts with other components can also be used. The mechanical resistance of an article can further be increased if a selectively adjusted compressive stress profile is built up within the article. This can be achieved by mono- or multistage ion exchange processes.

By replacing small ions with large ions or by thermal tempering, a compressive stress is created in the corresponding zone, which drops from the surface of the glass article towards the center. The maximum compressive stress is just below the glass surface and is also referred to as CS (compressive stress). CS is a stress and is expressed in units of MPa. The depth of the compressive stress layer is abbreviated as "DoL" and is given in the unit $\mu$m. Preferably, CS and DoL are measured using the FSM-60LE apparatus from Orihara.

In one embodiment, CS is greater than 100 MPa. Further preferably, CS is at least 200 MPa, at least 250 MPa, or at least 300 MPa. More preferably, CS is at most 1,000 MPa, at most 800 MPa, at most 600 MPa, or at most 500 MPa. Preferably, CS is in a range from >100 MPa to 1,000 MPa, from 200 MPa to 800 MPa, from 250 MPa to 600 MPa, or from 300 MPa to 500 MPa.

In one embodiment, the glass article is thermally toughened. Thermal toughening is typically achieved by rapid cooling of the hot glass surface. Thermal toughening has the advantage that the compressive stress layer can be formed deeper (larger DoL) than with chemical toughening. This makes the glasses less susceptible to scratching, since the compressive stress layer cannot be penetrated as easily with a scratch as with a thinner compressive stress layer.

The glasses or glass articles can, for example, be subjected to a thermal tempering process after a melting, shaping, annealing/cooling process and cold post-processing steps. In this process, glass bodies (e.g. a previously described glass article or a preliminary product), for example flat glass, are preferably fed horizontally or suspended into a device and rapidly heated to a temperature up to a maximum of 150 °C above the transformation temperature $T_G$. The surfaces of the glass body are then rapidly cooled, for example by blowing cold air through a nozzle system. As a result of the rapid cooling of the glass surfaces, they are frozen in an expanded network, while the interior of the glass body cools slowly and has time to contract more. This creates a compressive stress in the surface layer and a tensile stress in the interior. The amount of compressive stress depends on various glass parameters such as $CTE_{glass}$ (average linear coefficient of thermal expansion below Tg), $CTE_{liquid}$ (average linear coefficient of thermal expansion above Tg), strain point, softening point, Young's modulus and also on the amount of heat transfer between the cooling medium and the glass surface as well as the thickness of the glass bodies.

Preferably, a compressive stress of at least 50 MPa is generated. As a result, the flexural strength of the glass bodies can be doubled to tripled compared to non-toughened glass. In an embodiment, the glass is heated to a temperature of 750 to 800°C and tempered fast in as stream of cold air. Optionally, the blowing pressure may be from 1 to 16 kPa. With the glasses or glass articles described herein, values of compressive stress of 50 to 250 MPa, in particular 75 to 200 MPa, for example, are achieved on commercially available systems.

In one embodiment, the glass article has a compressive stress layer with a compressive stress of at least 50 MPa, in particular at least 75 MPa, at least 85 MPa or at least 100 MPa. The glass article may have a compressive stress layer on one, two or all of its surfaces. The compressive stress of the compressive stress layer may be limited to at most 250 MPa, at most 200 MPa, at most 160 MPa or at most 140 MPa. These compressive stress values may be present, in particular, in thermally toughened glass articles.

In one embodiment, the depth of the compressive stress layer of the glass article is at least 10 $\mu$m, at least 20 $\mu$m, at least 30 $\mu$m, or at least 50 $\mu$m. In certain embodiments, this layer may even be at least 80 $\mu$m, at least 100 $\mu$m, or at least 150 $\mu$m. Optionally, the DoL is limited to at most 2,000 $\mu$m, at most 1,500 $\mu$m, at most 1,250 $\mu$m, or at most 1,000 $\mu$m. In particular, the DoL can be from 10 $\mu$m to 2,000 $\mu$m, from 20 $\mu$m to 1,500 $\mu$m, or from 30 $\mu$m to 1,250 $\mu$m. In one embodiment, the glass article is thermally toughened with a DoL of at least 300 $\mu$m, at least 400 $\mu$m or at least 500 $\mu$m. Optionally, the DoL may be at most 2,000 $\mu$m, at most 1,500 $\mu$m, or at most 1,250 $\mu$m. In one embodiment, the DoL is from 300 $\mu$m to 2,000 $\mu$m, from 400 $\mu$m to 1,500 $\mu$m, or from 500 $\mu$m to 1,250 $\mu$m.

**Embodiments**

The present invention relates to a glass that is resistant in several respects. Particularly resistant glass is especially useful where the glass is exposed to special requirements. This is the case, for example, in extreme environments. Extreme environments are in particular areas of application in which special resistance, durability and safety are required, e.g. areas requiring explosion protection.

In one embodiment, the invention relates to a glass article with special suitability for use in extreme environments. The article may be a sheet, disc, tube, rod, ingot or block. In preferred embodiments, the article is in the form of a sheet or a disc.

The glass article consists of a glass having a transmission throughout the wavelength range of from 207 nm to 222 nm of at least 60% (measured at a thickness of 1 mm), wherein the glass is a borosilicate glass having a total platinum content of not more than 3.5 ppm, and a hydrolytic resistance characterized by an extracted $Na_2O$ equivalent in $\mu$g per g glass determined according to ISO 719 of not more than 250 $\mu$g/g, optionally wherein the glass article has a thickness of at least 0.3 mm, in particular at least 3 mm and/or up to 20 mm.

In extreme environments, it may be useful to provide a certain minimum thickness for the glass article, since thicker glasses are mechanically more stable than thinner glasses. However, thicker glass absorbs a greater portion of the UV radiation entering the glass, resulting in the generation of heat. In environments with highly flammable materials, high heat generation can be problematic. A glass article with low induced extinction at 200 nm and/or 254 nm offers the advantage that transmission remains high for the wavelengths under consideration, even after extended use, and extreme heat generation is avoided.

According to the invention, the glass article can also be used in a UV lamp for disinfecting surfaces in extreme environments. In one embodiment, the glass article is used in a UV lamp (particularly as a cover) that is used to disinfect a site of action. The site of action may be an object that is touched by many people, for example a handle, in particular a door handle. The UV lamp can, for example, be aligned in such a way that it applies UV radiation to the site of action. In this case, a certain proximity to the site of action cannot be avoided. Accordingly, there is a risk here that the glass article will be damaged by impacts. This results in a need for mechanical resistance. The mechanical resistance can be improved by a large thickness of the glass article, which, however, reduces the transmission of the article and greatly increases the heating of the glass during operation of the UV lamp. Excessive heating should be avoided, which in turn is positively influenced by very good transmission and low induced extinction. Excessively high temperatures impair safety due to the risk of user burns or explosions. In principle, the risk of burns can be reduced by greater distance, but this must be compensated with greater radiation intensity with the disadvantage again of stronger heat generation.

The disclosure also relates to a UV lamp and the use of the glass article in a UV lamp for disinfection, in particular in extreme environments, in particular for disinfecting sites of action, e.g. those touched by many people. It has proven advantageous to maintain a minimum distance between the surface to be disinfected and the glass article of 5 cm, in particular 7.5 cm or 10 cm. When using the glass article described herein, a power density of at least 1.0 mW/cm$^2$, at least 1.5 mW/cm$^2$, at least 2.5 mW/cm$^2$, at least 3.0 mW/cm$^2$ or at least 3.5 mW/cm$^2$ can be set at the site of action. The site of action is the surface to be disinfected. Optionally, the power density is at most 20 mW/cm$^2$, at most 15 mW/cm$^2$ or at most 10 mW/cm$^2$. In particular, the power density is the power that can be measured at the site of action as UV radiation, in particular UV-C radiation, mediated by the UV lamp. Preferably, the site of action is periodically disinfected. This means that the site of action is not irradiated continuously, but only intermittently. For example, an irradiation interval can be triggered by touch, presence or actuation by the user. For example, an irradiation interval may be at least 1 second, at least 5 seconds, at least 10 seconds, or at least 20 seconds. Optionally, an irradiation interval lasts at most 10 minutes, at most 5 minutes, at most 2 minutes, or at most 1 minute.

In one embodiment, the UV lamp and/or the glass article has a heat-optimized structure, wherein the thickness of the glass article and the UV transmission of the glass article are chosen in such a way that when a site of action 70 mm away from the glass article (disposed on the opposite side of the article with respect to the light source) is irradiated with a medium pressure mercury lamp at 120 W/cm and an arc length of 4 cm (e.g. Philips HOK 4/120) at a UVC power density of 17,27 mW/cm$^2$ for a duration of 5 seconds at an ambient temperature of 20°C, no temperature at the surface of the glass article facing the site of action exceeds 45°C. In one embodiment, the radiation passes perpendicularly through the glass article, i.e., the light enters the glass article substantially perpendicular to the surface facing the light source and/or the light exits the glass article substantially perpendicular to the surface of the glass article facing the site of action. In particular, no temperature exceeds a value of 42.5°C, 40°C or 37.5°C. In one embodiment, said temperature limits are not exceeded even after 10 seconds, 20 seconds, 30 seconds, 45 seconds, 60 seconds, 90 seconds, 120 seconds, 150 seconds or 180 seconds of irradiation. The property describes how strongly the glass article heats up when irradiated vertically with commonly used UV light sources. It is achieved that a UV lamp with a lamp cover made of the glass article does not become dangerously hot. UVC power density refers to the power density imparted by radiation in the UVC range (280 to 200 nm). Medium-pressure mercury lamps also emit light at other wavelengths, which are not taken into account here when considering UVC power density. The measurement is performed under ambient atmosphere. For clarification: the described property does not limit the UV lamp or the application of the glass article to medium-pressure mercury lamps.

In one embodiment, the glass article meets the requirements for the fracture pattern according to DIN EN 12150-1:2020-07. A whole article or a part of an article can be examined; in deviation from the specified standard, the article can be smaller than indicated there, as long as the area to be considered is exceeded. The area to be considered for the breakage pattern can be in particular 40 mm × 40 mm or 25 mm × 25 mm. In one embodiment, the glass article breaks into not less than 25 pieces, in particular not less than 30 pieces or not less than 40 pieces, under the above conditions. It is advantageous for the article to break into many pieces, since in the event of breakage the risk of injury is low if the pieces are small.

The fracture pattern can be influenced, for example, by the choice of glass composition, cooling condition (thermal shrinkage), by adjusting stresses in the glass and/or by tempering the article.

In one embodiment, the invention relates to a glass article consisting of a glass having a transmission throughout the wavelength range of from 207 nm to 222 nm of at least 60% (measured at a thickness of 1 mm), wherein the glass is a borosilicate glass having a total platinum content of not more than 3.5 ppm, and a hydrolytic resistance characterized by an extracted $Na_2O$ equivalent in $\mu$g per g glass determined according to ISO 719 of not more than 250 $\mu$g/g, further wherein the glass article has a thickness of at least 0.3 mm, in particular at least 3 mm and/or up to 20 mm, further wherein the article has a compressive stress on at least one surface of at least 50 MPa and a fracture pattern characterized by fracture of an area of 40 mm × 40 mm into not less than 25 pieces determined according to DIN EN 12150-1.

**Examples**

Example 1

All glasses were melted under reducing conditions in the quartz glass crucible and homogenized with a quartz glass stirrer. All melts were placed in the cooling furnace at 465°C and cooled to room temperature at a rate of 30 K/h.

The melts "glass No. 1" was melted at 1610° C, refined at 1620° C for 60 min, then stirred at the same temperature for 30 min, and then left to stand for 120 min at 1620° C, so that the glass was as bubble-free as possible.

After casting, the Pt-free variant ("Glass No. 1") resulted in clear glass cast blocks.

In addition to a chemical analysis of the glasses, the transmission curves of the glasses were recorded at at least two different points on the cast blocks. In order to be able to calculate the transmission curves for the same sample thickness, the refractive index was also determined as a function of the wavelength.

The following table shows an exemplary glass in accordance with this disclosure in mol%.

| Component | Glass No. 1* |
|---|---|
| $SiO_2$ | 70.07 |
| $Al_2O_3$ | 3.27 |
| $B_2O_3$ | 18.15 |
| $Li_2O$ | 1.55 |
| $Na_2O$ | 2.43 |
| $K_2O$ | 1.02 |
| CaO | 0.63 |
| BaO | 0.47 |
| $F^-$ | 2.27 |
| $Cl^-$ | 0.14 |
| Pt | 0.00 |
| Sum | 100.00 |
| *Glass No. 1 comprised about 4.4 ppm $Fe_2O_3$ and 3.8 ppm $TiO_2$. | |

Two "comparative glasses" of the same composition as "Glass No. 1" were produced comprising:

- "Comparative glass 1 (C1)": 7.9 ppm $Fe_2O_3$ and 8.3 ppm $TiO_2$ and 3.5 ppm Pt.
- "Comparative glass 2 (C2)": 3.3 ppm $Fe_2O_3$ and 20.9 ppm $TiO_2$ and 3.8 ppm Pt.

| Properties of glass no.1 | |
|---|---|
| CTE [ppm/K] | 4.14 |

(continued)

| Properties of glass no.1 | |
|---|---|
| $T_g$ [°C] | 443 |
| $T_4$ [°C] | 1085 |
| CTE $\times$ $T_4$ [$10^{-6}$] | **4491.9** |
| H equivalent $Na_2O$ [µg/g] | 101 |
| $n_d$ | 1.4761 |
| T @ 200 nm, 0 h DUV [T in % / d (mm)] | 68.1 / 0.73 |
| T @ 200 nm, 48 h DUV [T in % / $\alpha(\lambda)$] | 63.5 / 0.070 |
| T @ 200 nm, 96 h DUV [T in % / $\alpha(\lambda)$] | 58.4 / 0.154 |

*UV-transmission tests with Glass No. 1*

The UV-transmission of Glass No. 1 was tested at different thicknesses and wavelengths. At a thickness of 0.34 mm the following transmission was measured:

- 200 nm - 73.5%

- 207 nm - 77.5%

- 222 nm - 83.0%

At a thickness of 1 mm the following transmission was measured:

- 200 nm - 62.2%

- 207 nm - 67.5%

- 222 nm - 77.0%

Glass No. 1 was then compared to comparative glasses C1 and C2, with a Pt-contamination of 3.5 and 3.8 ppm, respectively.
The transmission at 200 nm was reduced from 60-65% to about 50% for glass C1 and to about 20-30% for glass C2, as compared to glass No. 1, showing the high influence of Pt-contamination, as well as an impact on the transmission by other contaminations such as iron and titanium.

*Production of UV-lamp and/or UV-LED-lamp*

The UVC-transparent glass No. 1 was used to produce a UVC-LED-lamp, by using the glass as cover in the LED-package. The LED package had a package size of 3.5 $\times$ 3.5mm.
Further features of the LED-package:

- Substrate: AIN with cavity (height ~ 1.0mm)
- Window thickness: 0.3mm (flat)
- Solder material: Au/Sn, Au/Ni

Furthermore, a UVC-transparent encapsulation material was used to further protect and cover the LED. Such encapsulation material may be copolymers of methyl methacrylate and acyloximino methacrylate ester. In this example poly-(methyl methacrylate-co-3-methacryloyl-oximino-2-butanone) was used.
Because of the advantageous thermic properties, the glass cover could be frit sealed with a laser to the package surface in order make the UVC-LED-lamp autoclavable even at elevated gas-pressures and to be useable in environments which comprise elevated humidity or gas-pressures.

*Energy Efficacy Test* as *UV-LED-cover*

The LED-lamp was compared with conventional LED-lamps. It could be shown that the LEDs made using the invention are about 30% more energy efficient than conventional UV-lamps.

*MRSA-eradication test*

A surface with MRSA-CFUs (colony forming units) were irradiated by UVC-LED-lamps of the present invention. 2,500 $\mu$W·s/cm$^2$ of UVC at 200 nm were applied for 10 minutes. After a UVC-treatment for 10 minutes less than 1% of MRSA - CFUs could be identified.

Thus, glass No. 1 is a glass with high UV-transmission and a hydrolytic resistance characterized by an equivalent amount of Na$_2$O extracted in water at 98°C of not more than 20, 25, 30, 50, 100, 180 and/or 250 $\mu$g/g.

<u>Example 2</u>

Further inventive glass-compositions with high UV-transmission and a hydrolytic resistance are depicted in the following table. The content of the components is listed in mol%. Further physical properties of the glasses are listed as well.

| | No. 1 | No. 2 | No. 3 | No. 4 | No. 5 | No. 6 |
|---|---|---|---|---|---|---|
| **SiO$_2$** | 70.07 | 71.01 | 71.33 | 71.88 | 71.20 | 71.85 |
| **Al$_2$O$_3$** | 3.27 | 3.94 | 4.15 | 3.91 | 3.97 | 4.16 |
| **B$_2$O$_3$** | 18.15 | 14.60 | 14.37 | 14.21 | 14.24 | 14.17 |
| **Li$_2$O** | 1.55 | 1.71 | 1.66 | 1.57 | 1.72 | 1.26 |
| **Na$_2$O** | 2.43 | 2.67 | 2.34 | 2.36 | 2.98 | 3.05 |
| **K$_2$O** | 1.02 | 1.06 | 1.23 | 1.06 | 1.07 | 1.06 |
| **CaO** | 0.62 | 0.56 | 0.74 | 0.56 | 0.56 | 0.56 |
| **SrO** | 0.0 | 0.02 | 0.01 | 0.01 | 0.02 | 0.01 |
| **BaO** | 0.47 | 1.22 | 1.25 | 1.25 | 1.25 | 1.03 |
| **F$^-$** | 2.27 | 2.99 | 2.72 | 3.03 | 2.77 | 2.65 |
| **Cl$^-$** | 0.14 | 0.23 | 0.20 | 0.16 | 0.23 | 0.19 |
| **Sum(*)** | **100.00** | **100.00** | **100.00** | **100.00** | **100.00** | **100.00** |
| **$\Sigma$ R$_2$O** | 5.00 | 5.43 | 5.23 | 5.00 | 5.76 | 5.37 |
| **$\Sigma$ RO** | 1.09 | 1.80 | 2.01 | 1.82 | 1.82 | 1.61 |
| **$\Sigma$ R$_2$O + $\Sigma$ RO** | 6.09 | 7.23 | 7.23 | 6.82 | 7.59 | 6.98 |
| **B$_2$O$_3$ / $\Sigma$ R$_2$O** | 3.63 | 2.69 | 2.75 | 2.84 | 2.47 | 2.64 |
| **B$_2$O$_3$ / $\Sigma$ RO** | 16.59 | 8.12 | 7.16 | 7.80 | 7.81 | 8.82 |
| **B$_2$O$_3$ / BaO** | 38.68 | 11.93 | 11.48 | 11.37 | 11.41 | 13.72 |
| **B$_2$O$_3$ / ($\Sigma$ RO + $\Sigma$ R$_2$O)** | 2.98 | 2.02 | 1.99 | 2.08 | 1.88 | 2.03 |
| **B$_2$O$_3$ / (SiO$_2$ + Al$_2$O$_3$)** | 0.25 | 0.19 | 0.19 | 0.19 | 0.19 | 0.19 |
| **$\Sigma$ R$_2$O / $\Sigma$ RO** | 4.57 | 3.02 | 2.61 | 2.74 | 3.16 | 3.35 |
| **Transmission @200 nm d=1mm [%]** | 63.7 | 61.2 | 64.6 | 62.7 | 63.0 | 64.5 |
| **Transmission @254 nm d=1mm [%]** | 87.4 | 86.4 | 87.4 | 86.2 | 84.4 | 86.1 |
| **CTE [ppm/K]** | 4.14 | 4.29 | 4.22 | 4.1 | 4.36 | 4.3 |
| **T$_g$ [°C]** | 443 | 462 | 469 | 468 | 466 | 466 |
| **T$_4$ [°C]** | 1085 | 1110 | 1121 | 1135 | 1099 | 1137 |

(continued)

|  | No. 1 | No. 2 | No. 3 | No. 4 | No. 5 | No. 6 |
|---|---|---|---|---|---|---|
| CTE*$T_4$ | 0.0045 | 0.0048 | 0.0047 | 0.0047 | 0.0048 | 0.0049 |
| H equ. $Na_2O$ [$\mu$g/g] (raw glass) | 101 | 16 | 19 | 17 | 23 | 30 |
| H equ. $Na_2O$ [$\mu$g/g] (phase separated) | 245 | 21 | 22 | 16 | 24 | 19 |
| (*) When produced in large scale, the inventive glasses 1 to 6 may comprise Pt below the detection limit (less than 1 ppm); less than 5 ppm $Fe_2O_3$ and less than 7 ppm $TiO_2$. | | | | | | |

The following table shows the solarisation resistance (induced absorbance) after exposure to a deuterium lamp for 48 and 96 h, respectively. The transmission was measured at a glass-thickness of 0.7 to 0.75 mm.

| induced extinction | No. 1 | No. 2 | No. 3 | No. 4 | No. 5 | No. 6 |
|---|---|---|---|---|---|---|
| 200nm, 48h | 0.070 | 0.018 | 0.053 | 0.031 | 0.022 | 0.026 |
| 200nm, 96h | 0.154 | 0.038 | 0.095 | 0.031 | 0.030 | 0.020 |

The following table shows rounded transmission-values for some glasses after exposure to a deuterium lamp after 48 and 96 h, respectively.

| Transmission [%] | No. 1 | No. 2 | No. 3 | No. 4 | No. 5 | No. 6 |
|---|---|---|---|---|---|---|
| 200nm, 48h | 63.5 | 66.4 | 65.8 | 67.4 | 68.5 | 69.6 |
| 200nm, 96h | 58.4 | 65.1 | 63.1 | 67.4 | 67.9 | 70.0 |

Detailed description of the figures

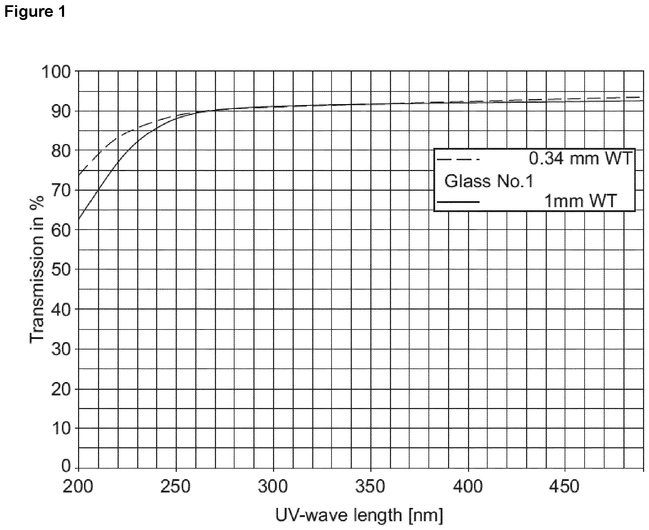

Figure 1 - Transmission curves of inventive "glass no.1" at different thicknesses (1 mm and 0.34 mm) and different UV-wavelengths.

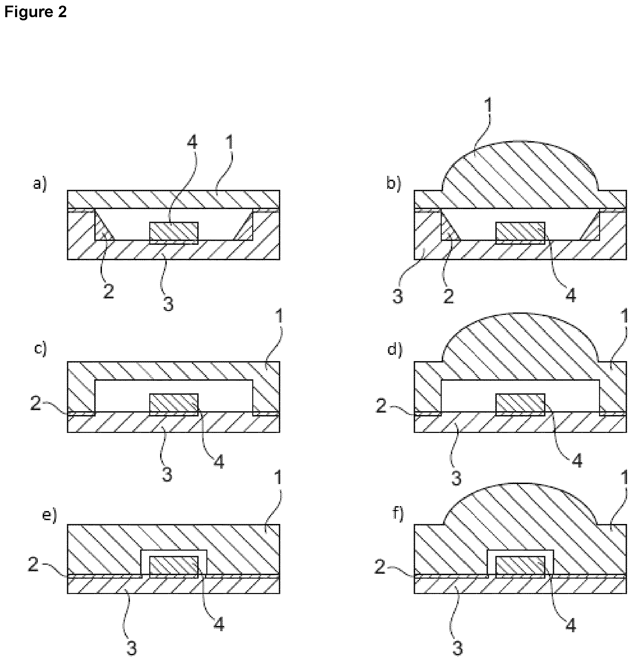

Figure 2 - Potential uses of the UV-transparent glasses in different LED-packages a) to f). The form of the glass lens [1] allows the focusing or dispersion of the UV-light, depending on the specific application. Furthermore, the cover glass [1] may surround the UV-source (e.g. an UV-LED) [4] so that UV-light is emitted also laterally (cf. figure 2 c) to f)). Reflective elements at the back of the casing [3] may improve the light emitting efficacy. As casing [3] an aluminium nitride ceramic - (AlN ceramic) with high thermal conductivity may be used. The LED [4] and the glass cover [1] may be metal soldered [2] to the casing [3].

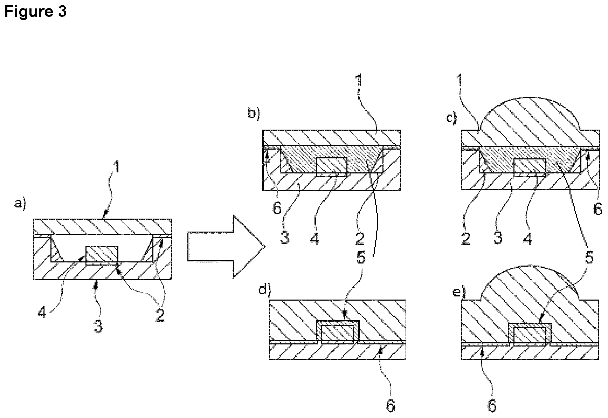

Figure 3 - Instead of metal soldering [2] the glass to the casing [3] (see figure 3, a)), the UV-transparent glasses [1] of the present invention may also be attached to the casing via laser frit sealing [6] (see figure 3, b) to e)). The LED [4], which may be metal soldered [2] to the casing [3], may be additionally fully encapsulated in transparent encapsulation material [5]. Such encapsulation material may be copolymers of methyl methacrylate and acyloximino methacrylate ester. In the examples poly-(methyl methacrylate-co-3-methacryloyl-oximino-2-butanone) was used. Due to the laser frit sealing [6] the LED-elements are then fully protected against environmental influences and, thus, this setup is most suitable for harsh environmental conditions, especially when strong acidic cleaners and/or disinfectants are used regularly. Again, as casing [3] an aluminium nitride ceramic - (AlN ceramic) with high thermal conductivity may be used. Again, reflective elements at the back of the casing [3] may improve the light emitting efficacy.

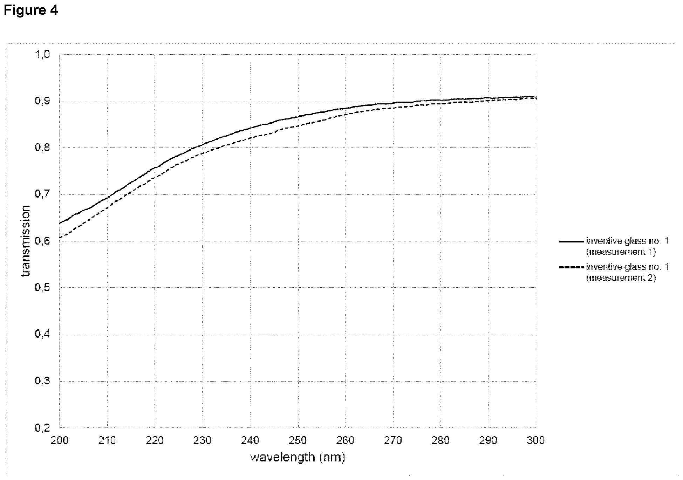

Figures 4 to 6 - Transmission curves of the different glasses. Transmission at 200 nm was reduced from 60-65% (inventive "glass no. 1") to about 50% ("comparative glass 1") and 20-30% ("comparative glass 2"), indicating the great influence on UV-transmission by platinum and other contaminations.

Figure 4 - Transmission curves of inventive "glass no. 1", which is free of Pt and has low contents of iron and titanium (made according to example 1). Two measurements at different points of the same glass cast block where made. Variations in transmission measurements of the same glass are due to inhomogeneities of the cast blocks.

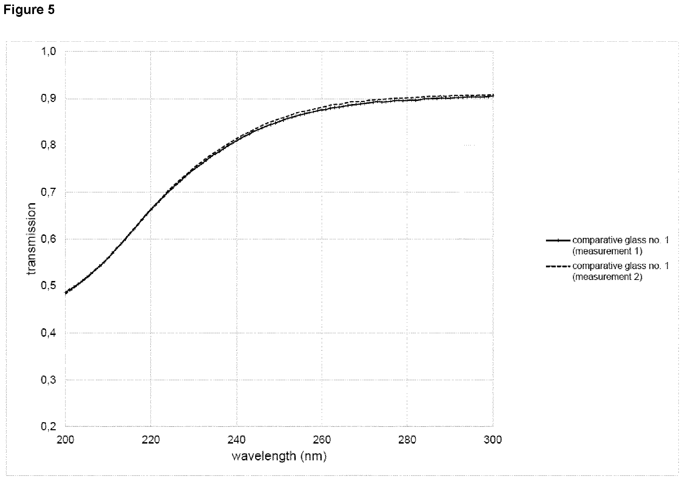

Figure 5 - Transmission curves of "comparative glass 1", which has a content of 3.5 ppm platinum, 7.9 ppm iron and 8.3 ppm titanium. Two measurements at different points of the same glass cast block where made. Variations in transmission measurements of the same glass are due to inhomogeneities of the cast blocks.

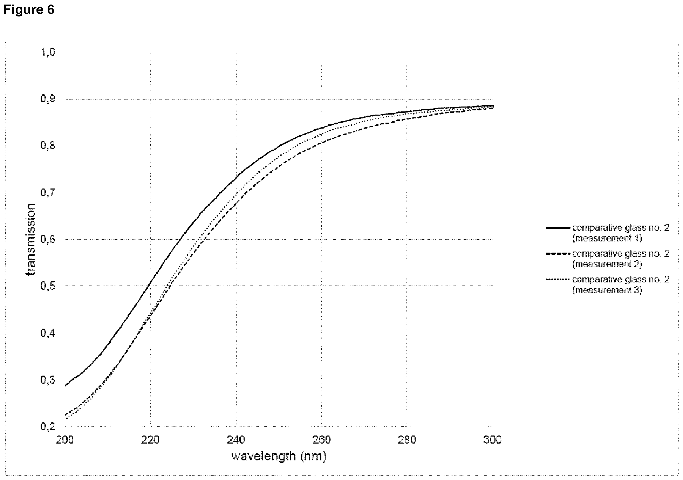

Figure 6 - Transmission curves of "comparative glass 2" has a content of 3.8 ppm platinum, 3.3 ppm iron and 20.9 ppm titanium. "comparative glass 2" was measured three times at different points of the same glass cast block. Variations in transmission measurements of the same glass are due to inhomogeneities of the cast blocks.

**Claims**

1. Method for eradicating Methicillin-resistant *Staphylococcus aureus* (MRSA), the method comprising exposing the MRSA to germicidal UV light within the wavelength range of from 207 nm to 222 nm, wherein the UV light is irradiated by a UV lamp having a lamp cover made of a borosilicate glass having a transmission throughout the wavelength range of from 207 nm to 222 nm of at least 60% measured at a thickness of 1 mm and having a total platinum content of less than 3.5 ppm and a hydrolytic resistance **characterized by** an extracted $Na_2O$ equivalent in $\mu g$ per g glass determined according to ISO 719 of not more than 250 $\mu g/g$;

   wherein the glass comprises the following components in the indicated amounts (in mol%):

   | Component | Content [mol%] |
   |---|---|
   | $SiO_2$ | 60 to 78 |
   | $Al_2O_3$ | 0 to 10 |
   | $B_2O_3$ | 12 to 24 |
   | $Li_2O$ | 0 to 3 |
   | $Na_2O$ | 0 to 6 |
   | $K_2O$ | 0 to 4 |
   | MgO | 0 to 6 |
   | CaO | 0 to 6 |
   | SrO | 0 to 4 |
   | BaO | 0 to 4 |
   | $F^-$ | 0 to 6 |
   | $Cl^-$ | 0 to 0.5 |
   | $R_2O$ | 3.5 to 10 |
   | RO | 0 to 6 |

   wherein "$R_2O$" refers to the alkali metal oxides $Li_2O$, $Na_2O$ and $K_2O$; and "RO" denotes the alkaline earth metal oxides MgO, CaO, BaO and SrO;
   and wherein the sum of the contents of Pt, $TiO_2$ and $Fe_2O_3$ is below 13.5 ppm.

2. Method of any of claim 1, wherein the exposed MRSA resides on a UV-sensitive material, which is sensitive to UV-radiation above 222 nm.

3. Method according to any of the previous claims, wherein the UV-sensitive material is a biological tissue surface such as the eye or skin of an animal, wherein the animal is selected from an insect, invertebrate, vertebrate, mammal and/or human.

4. Method according to any of the previous claims, wherein the eradication of MRSA after treatment is > 99% according to BS ISO 22196:2011-08-31.

5. Method according to any of the previous claims, wherein the UV-exposure of the MRSA is in the range from 2,000 to 8,000 microwatt seconds per square centimeter ($\mu W \cdot s/cm^2$).

6. Method according any of the previous claims, wherein all or part of the cover of the UV lamp is shaped in form of a lens.

7. Glass having a transmission throughout the wavelength range of from 207 nm to 222 nm of at least 60% measured at a thickness of 1 mm, wherein the glass is a borosilicate glass having a total platinum content of not more than 3.5 ppm, and a hydrolytic resistance **characterized by** an extracted $Na_2O$ equivalent in $\mu g$ per g glass determined according to ISO 719 of not more than 250 $\mu g/g$;

wherein the sum of the contents of Pt, $TiO_2$ and $Fe_2O_3$ is below 13.5 ppm;
wherein the glass comprises the following components in the indicated amounts (in mol%):

| Component | Content [mol%] |
|---|---|
| $SiO_2$ | 60 to 78 |
| $Al_2O_3$ | 0 to 10 |
| $B_2O_3$ | 12 to 24 |
| $Li_2O$ | 0 to 3 |
| $Na_2O$ | 0 to 6 |
| $K_2O$ | 0 to 4 |
| MgO | 0 to 6 |
| CaO | 0 to 6 |
| SrO | 0 to 4 |
| BaO | 0 to 4 |
| $F^-$ | 0 to 6 |
| $Cl^-$ | 0 to 0.5 |
| $R_2O$ | 3.5 to 10 |
| RO | 0 to 6 |

wherein "$R_2O$" refers to the alkali metal oxides $Li_2O$, $Na_2O$ and $K_2O$; and "RO" denotes the alkaline earth metal oxides MgO, CaO, BaO and SrO.

8. Glass according to claim 7, with

- a transmission of at least 60% at 200 nm and/or at least 85% at wavelengths [$\lambda$] of 260 nm, 280 nm and/or 310 nm, measured at a thickness of 1 mm;
- and/or
- the glass having a refractive index $n_d$ of from 1.450 to 1.580.

9. Glass according to claim 7 or 8, wherein

- a total content of one or more UV-blocking impurities is below 10 ppm,
- optionally, UV-blocking impurities are selected from lead, cadmium, mercury, rhodium, hexavalent chromium, iron, titanium, and any combination thereof.

10. Glass according to any of claims 7 to 9, wherein the total platinum content is below 1.0 ppm.

11. Glass article made of a glass according to any of claims 7 to 10, having thickness of at least 0.3 mm, in particular at least 3 mm and/or up to 20 mm.

12. Glass article according to claim 11, wherein the article is thermally or chemically toughened, particularly having a compressive stress on at least one surface of at least 50 MPa.

13. Glass article according to claim 11 or 12, having a fracture pattern **characterized by** fracture of an area of 40 mm × 40 mm into not less than 25 pieces determined according to DIN EN 12150-1.

14. Use of the glass according to any of claims 7 to 13 as a hermetically sealing lens cap for an UV-LED-module, e.g. for applications selected from the group of water disinfection, analytical instrumentation such as HPLC, spectrometers, water monitoring sensors, air purification, air disinfection, surface disinfection such as keyboard disinfection, escalator handrail UV sterilizer, cytometry, molecular identification, protein analysis, biofilm treatment, curing, lithography, vegetable growth, skin cure such as psoriasis, vitiligo, itching, neurodermatitis, acne, actinic dermatitis, phototherapy, pityriasis rosea, germ detection, drug discovery, protein analysis, induction of skin vit-D3-production and/or sterilization.

15. Use of the glass article according to claim 14 or glass article according to one of claims 11 to 13, wherein the thickness of the glass article and the UV transmission of the glass article are chosen in such a way that when a site of action 70 mm away from the glass article, disposed on the opposite side of the article with respect to the light source, is irradiated with a medium pressure mercury lamp at 120 W/cm and an arc length of 4 cm at a UVC power density of 17.27 mW/cm$^2$ for a duration of 5 seconds at an ambient temperature of 20°C, no temperature at the surface of the glass article facing the site of action exceeds 45°C.

**Patentansprüche**

1. Verfahren zur Ausrottung von Methicillin-resistentem *Staphylococcus aureus* (MRSA), wobei das Verfahren Belichten von dem MRSA mit keimtötendem UV-Licht innerhalb des Wellenlängenbereichs von 207 nm bis 222 nm umfasst, wobei das UV-Licht ausgestrahlt wird von einer UV-Lampe mit einer Lampenabdeckung hergestellt aus einem Borosilikatglas mit einer Transmission durchgehend im Wellenlängenbereich von 207 nm bis 222 nm von mindestens 60% gemessen bei einer Dicke von 1 mm und mit einem Platingesamtgehalt von niedriger als 3,5 ppm und einer hydrolytischen Beständigkeit charakterisiert durch ein extrahiertes $Na_2O$-Äquivalent in µg pro g Glas bestimmt gemäß ISO 719 von nicht höher als 250 µg/g;
wobei das Glas die folgenden Komponenten in den angegebenen Mengen (in Mol-%) umfasst:

| Komponente | Gehalt [Mol-%] |
|---|---|
| $SiO_2$ | 60 bis 78 |
| $Al_2O_3$ | 0 bis 10 |
| $B_2O_3$ | 12 bis 24 |
| $Li_2O$ | 0 bis 3 |
| $Na_2O$ | 0 bis 6 |
| $K_2O$ | 0 bis 4 |
| MgO | 0 bis 6 |
| CaO | 0 bis 6 |
| SrO | 0 bis 4 |
| BaO | 0 bis 4 |
| $F^-$ | 0 bis 6 |
| $Cl^-$ | 0 bis 0,5 |
| $R_2O$ | 3,5 bis 10 |
| RO | 0 bis 6 |

wobei "$R_2O$" die Alkalimetalloxide $Li_2O$, $Na_2O$ und $K_2O$ betrifft; und "RO" die Erdalkalimetalloxide MgO, CaO, BaO und SrO bezeichnet;

und wobei die Summe der Gehalte an Pt, $TiO_2$ und $Fe_2O_3$ unter 13,5 ppm liegt.

2. Verfahren nach jedwedem von Anspruch 1, wobei sich der belichtete MRSA auf einem UV-empfindlichen Material, welches für UV-Strahlung über 222 nm empfindlich ist, befindet.

3. Verfahren gemäß einem der vorhergehenden Ansprüche, wobei das UV-empfindliche Material eine biologische Gewebeoberfläche wie das Auge oder die Haut von einem Tier ist, wobei das Tier aus einem Insekt, wirbellosen Tier, Wirbeltier, Säuger und/oder Menschen ausgewählt ist.

4. Verfahren gemäß einem der vorhergehenden Ansprüche, wobei die Ausrottung von MRSA nach Behandlung gemäß BS ISO 22196:2011-08-31 > 99% ist.

5. Verfahren gemäß einem der vorhergehenden Ansprüche, wobei die UV-Belichtung von dem MRSA im Bereich von 2.000 bis 8.000 Mikrowatt Sekunden pro Quadratzentimeter ($\mu W \cdot s/cm^2$) liegt.

6. Verfahren gemäß einem der vorhergehenden Ansprüche, wobei die gesamte oder ein Teil der Abdeckung der UV-Lampe in der Form einer Linse gestaltet ist.

7. Glas mit einer Transmission durchgehend im Wellenlängenbereich von 207 nm bis 222 nm von mindestens 60% gemessen bei einer Dicke von 1 mm, wobei das Glas ein Borosilikatglas mit einem Platingesamtgehalt von nicht höher als 3,5 ppm, und einer hydrolytischen Beständigkeit charakterisiert durch ein extrahiertes $Na_2O$-Äquivalent in $\mu g$ pro g Glas bestimmt gemäß ISO 719 von nicht höher als 250 $\mu g/g$ ist;

wobei die Summe der Gehalte an Pt, $TiO_2$ und $Fe_2O_3$ unter 13,5 ppm liegt;
wobei das Glas die folgenden Komponenten in den angegebenen Mengen (in Mol-%) umfasst:

| Komponente | Gehalt [Mol-%] |
|---|---|
| $SiO_2$ | 60 bis 78 |
| $Al_2O_3$ | 0 bis 10 |
| $B_2O_3$ | 12 bis 24 |
| $Li_2O$ | 0 bis 3 |
| $Na_2O$ | 0 bis 6 |
| $K_2O$ | 0 bis 4 |
| MgO | 0 bis 6 |
| CaO | 0 bis 6 |
| SrO | 0 bis 4 |
| BaO | 0 bis 4 |
| $F^-$ | 0 bis 6 |
| $Cl^-$ | 0 bis 0,5 |
| $R_2O$ | 3,5 bis 10 |
| RO | 0 bis 6 |

wobei "$R_2O$" die Alkalimetalloxide $Li_2O$, $Na_2O$ und $K_2O$ betrifft; und "RO" die Erdalkalimetalloxide MgO, CaO, BaO und SrO bezeichnet.

8. Glas gemäß Anspruch 7, mit

- einer Transmission von mindestens 60% bei 200 nm und/oder mindestens 85% bei Wellenlängen [$\lambda$] von 260 nm, 280 nm und/oder 310 nm, gemessen bei einer Dicke von 1 mm;
- und/oder
- wobei das Glas einen Brechungsindex $n_d$ von 1,450 bis 1,580 aufweist.

9. Glas gemäß Anspruch 7 oder 8, wobei

   - ein Gesamtgehalt von einer oder mehr UV-blockierenden Verunreinigungen unter 10 ppm liegt,
   - gegebenenfalls, UV-blockierende Verunreinigungen aus Blei, Cadmium, Quecksilber, Rhodium, sechswertigem Chrom, Eisen, Titan, und jedweder Kombination davon ausgewählt sind.

10. Glas gemäß einem der Ansprüche 7 bis 9, wobei der Platingesamtgehalt unter 1,0 ppm liegt.

11. Glasgegenstand hergestellt aus einem Glas gemäß einem der Ansprüche 7 bis 10, mit einer Dicke von mindestens 0,3 mm, insbesondere mindestens 3 mm und/oder bis zu 20 mm.

12. Glasgegenstand gemäß Anspruch 11, wobei der Gegenstand thermisch oder chemisch verfestigt ist, insbesondere mit einer Druckspannung auf mindestens einer Oberfläche von mindestens 50 MPa.

13. Glasgegenstand gemäß Anspruch 11 oder 12, mit einem Bruchbild charakterisiert durch einen Bruch einer Fläche von 40 mm × 40 mm zu nicht weniger als 25 Bruchstücken bestimmt gemäß DIN EN 12150-1.

14. Verwendung des Glases gemäß einem der Ansprüche 7 bis 13 als eine hermetisch versiegelnde Linsenkappe für ein UV-LED-Modul, z.B. für Verwendungen ausgewählt aus der Gruppe von Wasserdesinfektion, analytischer Geräteausstattung wie HPLC, Spektrometern, Wasserüberwachungssensoren, Luftreinigung, Luftdesinfektion, Oberflächendesinfektion wie Tastaturdesinfektion, Fahrtreppenhandlauf-UV-Sterilisierungsgerät, Zytometrie, Molekülidentifizierung, Proteinanalyse, Biofilmbehandlung, Härten, Lithografie, Pflanzenwachstum, Hautheilung wie Psoriasis, Vitiligo, Juckreiz, Neurodermitis, Akne, aktinische Dermatitis, Fototherapie, Pityriasis rosea, Keimnachweis, Wirkstoffentdeckung, Proteinanalyse, Induzieren der Herstellung von Vitamin D3 durch die Haut und/oder Sterilisierung.

15. Verwendung des Glasgegenstandes gemäß Anspruch 14 oder Glasgegenstand gemäß einem der Ansprüche 11 bis 13, wobei die Dicke des Glasgegenstandes und die UV-Transmission des Glasgegenstandes derart gewählt sind, dass, wenn ein Wirkort 70 mm entfernt von dem Glasgegenstand, welcher sich auf der gegenüberliegenden Seite des Gegenstandes in Bezug auf die Lichtquelle befindet, mit einer Mitteldruck-Quecksilberlampe bei 120 W/cm und einer Bogenlänge von 4 cm bei einer UVC-Leistungsdichte von 17,27 mW/cm$^2$ für eine Dauer von 5 Sekunden bei einer Umgebungstemperatur von 20°C bestrahlt wird, an der Oberfläche des Glasgegenstandes, welche dem Wirkort zugewandt ist, keine Temperatur 45°C überschreitet.

**Revendications**

1. Procédé d'éradication du Staphylococcus aureus résistant à la méthicilline (MRSA), le procédé comprenant l'exposition du MRSA à une lumière UV germicide dans la gamme de longueurs d'onde de 207 nm à 222 nm, dans lequel la lumière UV est irradiée par une lampe UV ayant un couvercle de lampe fait d'un verre borosilicate ayant une transmission dans toute la gamme de longueurs d'onde de 207 nm à 222 nm d'au moins 60 % mesurée à une épaisseur de 1 mm et ayant une teneur totale en platine de moins de 3,5 ppm et une résistance hydrolytique **caractérisée par** un équivalent $Na_2O$ extrait en μg par g de verre déterminé selon la norme ISO 719 ne dépassant pas 250 μg/g ;

   dans lequel le verre comprend les composants suivants dans les quantités indiquées (en mol%) :

| Composant | Teneur (mol%) |
|---|---|
| $SiO_2$ | 60 à 78 |
| $Al_2O_3$ | 0 à 10 |
| $B_2O_3$ | 12 à 24 |
| $Li_2O$ | 0 à 3 |
| $Na_2O$ | 0 à 6 |
| $K_2O$ | 0 à 4 |
| MgO | 0 à 6 |

(continued)

| Composant | Teneur (mol%) |
|-----------|---------------|
| CaO | 0 à 6 |
| SrO | 0 à 4 |
| BaO | 0 à 4 |
| F⁻ | 0 à 6 |
| Cl⁻ | 0 à 0,5 |
| $R_2O$ | 3,5 à 10 |
| RO | 0 à 6 |

dans lequel « $R_2O$ » désigne les oxydes de métaux alcalins $Li_2O$, $Na_2O$ et $K_2O$ ; et « RO » désigne les oxydes de métaux alcalino-terreux MgO, CaO, BaO et SrO ;
et dans lequel la somme des teneurs en Pt, $TiO_2$ et $Fe_2O_3$ est inférieure à 13,5 ppm.

2. Procédé selon la revendication 1, dans lequel le MRSA exposé réside sur un matériau sensible aux UV, qui est sensible à une radiation UV supérieure à 222 nm.

3. Procédé selon l'une quelconque des revendications précédentes, dans lequel le matériau sensible aux UV est une surface de tissu biologique telle que l'œil ou la peau d'un animal, l'animal étant choisi parmi un insecte, un invertébré, un vertébré, un mammifère et/ou un humain.

4. Procédé selon l'une quelconque des revendications précédentes, dans lequel l'éradication du MRSA après traitement est >99% selon la norme BS ISO 22196:2011-08-31.

5. Procédé selon l'une quelconque des revendications précédentes, dans lequel l'exposition du MRSA aux UV est comprise entre 2 000 et 8 000 microwatts secondes par centimètre carré ($\mu$W.s/cm²).

6. Procédé selon l'une quelconque des revendications précédentes, dans lequel tout ou partie du couvercle de la lampe UV est façonné en forme de lentille.

7. Verre ayant une transmission dans toute la gamme de longueurs d'onde de 207 nm à 222 nm d'au moins 60% mesurée à une épaisseur de 1 mm, dans lequel le verre est un verre borosilicate ayant une teneur totale en platine ne dépassant pas 3,5 ppm, et une résistance hydrolytique **caractérisée par** un équivalent $Na_2O$ extrait en $\mu$g par g de verre déterminé selon la norme ISO 719 ne dépassant pas 250 $\mu$g/g ;

dans lequel la somme des teneurs en Pt, $TiO_2$ et $Fe_2O_3$ est inférieure à 13,5 ppm ;
dans lequel le verre comprend les composants suivants dans les quantités indiquées (en mol%) :

| Composant | Teneur (mol%) |
|-----------|---------------|
| $SiO_2$ | 60 à 78 |
| $Al_2O_3$ | 0 à 10 |
| $B_2O_3$ | 12 à 24 |
| $Li_2O$ | 0 à 3 |
| $Na_2O$ | 0 à 6 |
| $K_2O$ | 0 à 4 |
| MgO | 0 à 6 |
| CaO | 0 à 6 |
| SrO | 0 à 4 |
| BaO | 0 à 4 |

(continued)

| Composant | Teneur (mol%) |
|-----------|---------------|
| F⁻ | 0 à 6 |
| Cl⁻ | 0 à 0,5 |
| $R_2O$ | 3,5 à 10 |
| RO | 0 à 6 |

dans lequel « $R_2O$ » désigne les oxydes de métaux alcalins $Li_2O$, $Na_2O$ et $K_2O$ ; et « RO » désigne les oxydes de métaux alcalino-terreux MgO, CaO, BaO et SrO.

8.  Verre selon la revendication 7, avec

    - une transmission d'au moins 60% à 200 nm et/ou d'au moins 85% à des longueurs d'onde [λ] de 260 nm, 280 nm et/ou 310 nm, mesurée à une épaisseur de 1mm ;
    - et/ou
    - le verre ayant un indice de réfraction $n_d$ de 1,450 à 1,580.

9.  Verre selon la revendication 7 ou 8, dans lequel

    - une teneur totale en une ou plusieurs impuretés bloquant les UV est inférieure à 10 ppm,
    - éventuellement, les impuretés bloquant les UV sont choisies parmi le plomb, le cadmium, le mercure, le rhodium, le chrome hexavalent, le fer, le titane et toute combinaison de ces éléments.

10. Verre selon l'une quelconque des revendications 7 à 9, dans lequel la teneur totale en platine est inférieure à 1,0 ppm.

11. Article en verre constitué d'un verre selon l'une quelconque des revendications 7 à 10, d'une épaisseur d'au moins 0,3 mm, en particulier d'au moins 3 mm et/ou jusqu'à 20 mm.

12. Article en verre selon la revendication 11, dans lequel l'article est trempé thermiquement ou chimiquement, en particulier ayant une contrainte de compression sur au moins une surface d'au moins 50 MPa.

13. Article en verre selon la revendication 11 ou 12, présentant un schéma de rupture **caractérisé par** la rupture d'une surface de 40 mm x 40 mm en au moins 25 morceaux déterminés selon la norme DIN EN 12150-1.

14. Utilisation du verre selon l'une quelconque des revendications 7 à 13 comme capuchon de lentille hermétiquement scellé pour un module UV-LED, par exemple pour des applications choisies dans le groupe de la désinfection de l'eau, de l'instrumentation analytique telle que HPLC, des spectromètres, des capteurs de surveillance de l'eau, de la purification de l'air, de la désinfection de l'air, de la désinfection des surfaces telle que la désinfection des claviers, de la stérilisation UV des rampes d'escaliers roulants, de la cytométrie, de l'identification moléculaire, l'analyse des protéines, le traitement des biofilms, le durcissement, la lithographie, la croissance végétale, le traitement de la peau comme le psoriasis, le vitiligo, les démangeaisons, la neurodermite, l'acné, la dermatite actinique, la photothérapie, le pityriasis rosé, la détection des germes, la découverte de médicaments, l'analyse des protéines, l'induction de la production de vit-D3 dans la peau et/ou la stérilisation.

15. Utilisation de l'article en verre selon la revendication 14 ou de l'article en verre selon l'une des revendications 11 à 13, dans laquelle l'épaisseur de l'article en verre et la transmission UV de l'article en verre sont choisies de telle sorte que lorsqu'un site d'action situé à 70 mm de l'article en verre, disposé sur le côté opposé de l'article par rapport à la source lumineuse, est irradié par une lampe à mercure moyenne pression à 120 W/cm et une longueur d'arc de 4 cm à une densité de puissance UVC de 17,27 mW/cm² pendant une durée de 5 secondes à une température ambiante de 20°C, aucune température à la surface de l'article en verre faisant face au site d'action ne dépasse 45°C.

**Figure 1**

**Figure 2**

**Figure 3**

**Figure 4**

**Figure 5**

**Figure 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 110240402 A
- US 5045509 A
- JP S6287433 A

**Non-patent literature cited in the description**

- **NARITA KOUJI et al.** *Disinfection and healing effects of 222-nm UVC light on methicillin-resistantStaphylococcus aureusinfection in mouse wounds*
- **KAIKI YUKI et al.** *Methicillin-resistant Staphylococcus aureus contamination of hospital-use-only mobile phones and efficacy of 222-nm ultraviolet disinfection*